# EUROPEAN PATENT APPLICATION

(11) **EP 4 647 420 A1**
(43) Date of publication of application: **12.11.2025**
(21) Application number: 25172034.8
(22) Date of filing: 23.04.2025
(51) Int. Cl.: C07C 211/61, H10K 50/15, H10K 85/60

(54) **COMPOUND FOR ORGANIC ELECTRONIC ELEMENT, ORGANIC ELECTRONIC ELEMENT USING THE SAME, AND AN ELECTRONIC DEVICE THEREOF**

(30) Priority: 08.05.2024 KR 20240060256
(71) Applicant: Duk San Neolux Co., Ltd., Chungcheongnam-do 31027 (KR)
(72) Inventor: SEO, Jun seok, 31027 Cheonan-si, Chungcheongnam-do (KR); OH, Hyun Ji, 31027 Cheonan-si, Chungcheongnam-do (KR)
(74) Representative: Isarpatent

(57) **Abstract**

The present invention provides a novel compound that can improve the luminous efficiency, stability, and lifespan of the element, a composition comprising the same and an organic electronic element using the same, and an electronic device thereof.

## Description

### BACKGROUND

### [Technical Field]

The present invention relates to compounds for organic electronic elements, organic electronic elements using the same, and an electronic device thereof.

### [Background Art]

In general, organic light emitting phenomenon refers to a phenomenon that converts electric energy into light energy by using an organic material. An organic electronic element using an organic light emitting phenomenon usually has a structure including an anode, a cathode, and an organic material layer interposed therebetween. Here, in order to increase the efficiency and stability of the organic electronic element, the organic material layer is often composed of a multi-layered structure composed of different materials, and for example, may include a hole injection layer, a hole transport layer, an emitting layer, an electron transport layer, an electron injection layer etc.

A material used as an organic material layer in an organic electronic element may be classified into a light emitting material and a charge transport material, such as a hole injection material, a hole transport material, an electron transport material, an electron injection material etc. depending on its function.

The current portable display market is trending toward larger displays, which in turn requires greater power consumption than that required by existing portable displays. Therefore, power consumption has become a very important factor for portable displays that have a limited power source called a battery, and efficiency and lifespan issues must also be addressed.

Efficiency, lifespan and driving voltage are related to each other, and when the efficiency is increased, the driving voltage is relatively decreased, and as the driving voltage is decreased, crystallization of organic materials due to Joule heating generated during driving decreases, and consequently, the lifespan tends to increase.

However, the efficiency cannot be maximized simply by improving the organic material layer. This is because, when the energy level and T1 value between each organic material layer, and the intrinsic properties (mobility, interfacial properties, etc.) of materials are optimally combined, long lifespan and high efficiency can be achieved at the same time.

That is, in order to sufficiently exhibit the excellent characteristics of the organic electronic element, a material for forming an organic material layer in an element such as a hole injection material, a hole transport material, a light emitting material, an electron transport material, an electron injection material, an emitting-auxiliary layer material should be supported by stable and efficient materials. However, such a stable and efficient organic material layer material for an organic electronic element has not been sufficiently developed yet. Therefore, the development of new materials continues to be required.

### DETAILED DESCRIPTION OF THE INVENTION

### [Summary]

In order to solve the problems of the background art described above, the present invention has revealed a compound having a novel structure, and that when the compound is applied to an organic electronic element, the luminous efficiency, stability and lifespan of the element are greatly improved.

Accordingly, it is an object of the present invention to provide a novel compound, an organic electronic element using the same, and an electronic device thereof.

### [Technical Solution]

The present invention provides a compound represented by Formula 1.

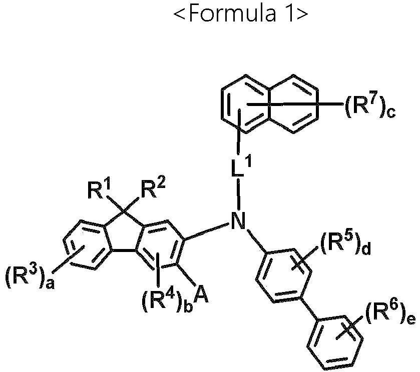

In another aspect, the present invention provides an organic electronic element comprising a compound represented by Formula 1 and an electronic device thereof.

### [Effects of the Invention]

By using the compound according to the present invention, it is possible to achieve a high luminous efficiency, a low driving voltage, and a high heat resistance of the element, and can greatly improve the color purity and lifespan of the element.

### [BRIEF DESCRIPTION OF THE DRAWINGS]

FIG. 1 to FIG. 3 illustrate an example of an organic electronic element according to the present invention.
FIG. 4 shows a Formula according to one aspect of the present invention.

### [DETAILED DESCRIPTION]

Hereinafter, some embodiments of the present invention will be described in detail. Further, in the following description of the present invention, a detailed description of known functions and configurations incorporated herein will be omitted when it may make the subject matter of the present invention rather unclear.

In addition, terms, such as first, second, A, B, (a), (b) or the like may be used herein when describing components of the present invention. Each of these terminologies is not used to define an essence, order or sequence of a corresponding component but used merely to distinguish the corresponding component from other component(s). It should be noted that if a component is described as being "connected", "coupled", or "connected" to another component, the component may be directly connected or connected to the other component, but another component may be "connected ", " coupled" or "connected" between each component.

As used in the specification and the accompanying claims, unless otherwise stated, the following is the meaning of the term as follows.

Unless otherwise stated, the term "halo" or "halogen", as used herein, includes fluorine(F), bromine(Br), chlorine(CI), or iodine(I).

Unless otherwise stated, the term "alkyl" or "alkyl group", as used herein, has a single bond of 1 to 60 carbon atoms, 1 to 30 carbon atoms, 1 to 25 carbon atoms, 1 to 18 carbon atoms, or 1 to 12 carbon atoms, and means saturated aliphatic functional radicals including a linear alkyl group, a branched chain alkyl group, a cycloalkyl group (alicyclic), an cycloalkyl group substituted with a alkyl or an alkyl group substituted with a cycloalkyl.

Unless otherwise stated, the term "alkenyl" or "alkynyl", as used herein, has double or triple bonds of 2 to 60 carbon atoms, 2 to 30 carbon atoms, 2 to 25 carbon atoms, 2 to 18 carbon atoms, 2 to 12 carbon atoms, but is not limited thereto, and includes a linear or a branched chain group.

Unless otherwise stated, the term "cycloalkyl", as used herein, means alkyl forming a ring having 3 to 60 carbon atoms, 3 to 30 carbon atoms, 3 to 25 carbon atoms, 3 to 18 carbon atoms, 3 to 12 carbon atoms, but is not limited thereto.

Unless otherwise stated, the term "alkoxyl group", "alkoxy group" or "alkyloxy group", as used herein, means an alkyl group bonded to oxygen radical, but is not limited thereto, and has 1 to 60 carbon atoms, 1 to 30 carbon atoms, 1 to 25 carbon atoms, 1 to 18 carbon atoms, or 1 to 12 carbon atoms.

Unless otherwise stated, the term "aryloxyl group" or "aryloxy group", as used herein, means an aryl group bonded to oxygen radical, but is not limited thereto, and has 6 to 60 carbon atoms, 6 to 30 carbon atoms, 6 to 25 carbon atoms, 6 to 18 carbon atoms, or 6 to 12 carbon atoms.

Unless otherwise specified, the terms "aryl group" and "arylene group" used in the present invention have 6 to 60 carbon atoms, 6 to 30 carbon atoms, 6 to 25 carbon atoms, 6 to 18 carbon atoms, or 6 to 12 carbon atoms, respectively, but are not limited thereto. In the present invention, an aryl group or arylene group refers to an aromatic group of a single ring or multiple rings, and comprises an aromatic ring formed by the bonding or reaction of adjacent substituents. For example, the aryl group may be a phenyl group, a biphenyl group, a fluorene group, or a spirofluorene group.

The prefix "aryl" or "ar" means a radical substituted with an aryl group. For example, an arylalkyl may be an alkyl substituted with an aryl, and an arylalkenyl may be an alkenyl substituted with aryl, and a radical substituted with an aryl has a number of carbon atoms as defined herein.

Also, when prefixes are named subsequently, it means that substituents are listed in the order described first. For example, an arylalkoxy means an alkoxy substituted with an aryl, an alkoxylcarbonyl means a carbonyl substituted with an alkoxyl, and an arylcarbonylalkenyl also means an alkenyl substituted with an arylcarbonyl, wherein the arylcarbonyl may be a carbonyl substituted with an aryl.

Unless otherwise stated, the term "heteroaryl group" or "heteroarylene group" as used in the present invention, means an aryl group or arylene group having 2 to 60 carbon atoms, 2 to 30 carbon atoms, 2 to 25 carbon atoms, 2 to 18 carbon atoms or 2 to 12 carbon atoms, each containing one or more heteroatoms, but is not limited thereto, and includes at least one of a single ring and multiple rings, and may be formed by bonding adjacent functional groups.

Unless otherwise stated, the term "heterocyclic group", as used herein, contains one or more heteroatoms, and has 2 to 60 carbon atoms, 2 to 30 carbon atoms, 2 to 25 carbon atoms, 2 to 18 carbon atoms, 2 to 12 carbon atoms, and comprises any one of a single ring or multiple ring, and may include heteroaliphatic ring and heteroaromatic ring. Also, the heterocyclic group may also be formed in conjunction with an adjacent group. Unless otherwise stated, the term "heteroatom", as used herein, represents at least one of N, O, S, P, or Si.

Additionally, "heterocyclic group" means a single ring, ring aggregate, fused multiple ring system, spiro compound, etc. containing a heteroatom. Also, compounds containing heteroatom groups such as SO₂, P=O, etc. instead of carbon forming a ring, such as the compounds below, can also be included in the heterocyclic group. For example, a "heterocyclic group" includes the following compound.

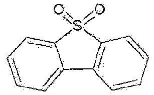

The term "aliphatic ring group" used in the present invention refers to cyclic hydrocarbons excluding aromatic hydrocarbons, and includes single rings, ring aggregates, fused multiple ring systems, spiro compounds, etc., and means a ring having 3 to 60 carbon atoms, 3 to 30 carbon atoms, 3 to 25 carbon atoms, 3 to 18 carbon atoms, 3 to 12 carbon atoms, but is not limited thereto. For example, even when benzene, an aromatic ring, and cyclohexane, a non-aromatic ring, are fused, it is an aliphatic ring.

Unless otherwise stated, the term "fluorenyl group", "fluorenylene group" or "fluorentriyl group" as used herein, means a monovalent, divalent or trivalent functional group, in which R, R' and R" are all hydrogen in the following structures, and the term "substituted fluorenyl group", "substituted fluorenylene group" or "substituted fluorentriyl group" means that at least one of the substituents R, R' and R" is a substituent other than hydrogen, and include those in which R and R' are bonded to each other to form a spiro compound together with the carbon to which they are bonded. In this specification, fluorenyl group, fluorenylene group, and fluorenetriyl group may all be referred to as fluorene groups, regardless of valence.

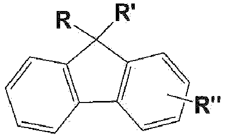

The term "spiro compound" used in the present invention has a 'spiro union', and a spiro union means a connection formed by 2 rings sharing only one atom. At this time, the atom shared between the 2 rings is called a 'spiro atom', and depending on the number of spiro atoms contained in a compound, they are called 'monospiro-', 'dispiro-', and 'trispiro-' compounds, respectively.

Unless otherwise stated, the term "aliphatic" as used herein means an aliphatic hydrocarbon having 1 to 60 carbon atoms, 1 to 30 carbon atoms, 1 to 25 carbon atoms, 1 to 18 carbon atoms or 1 to 12 carbon atoms, and "aliphatic ring" means an aliphatic hydrocarbon ring having 3 to 60 carbon atoms, 3 to 30 carbon atoms, 3 to 25 carbon atoms, 3 to 18 carbon atoms or 3 to 12 carbon atoms.

Unless otherwise stated, the term "ring", as used herein, means an aliphatic ring having 3 to 60 carbon atoms, 3 to 30 carbon atoms, 3 to 25 carbon atoms, 3 to 18 carbon atoms or 3 to 12 carbon atoms; or an aromatic ring having 6 to 60 carbon atoms, 6 to 30 carbon atoms, 6 to 25 carbon atoms, 6 to 18 carbon atoms, or 6 to 12 carbon atoms; or a heterocyclic having 2 to 60 carbon atoms, 2 to 30 carbon atoms, 2 to 25 carbon atoms, 2 to 18 carbon atoms, 2 to 12 carbon atoms, or a fused ring formed by the combination thereof, and includes a saturated or unsaturated ring.

Other hetero compounds or hetero radicals other than the above-mentioned hetero compounds include, but are not limited thereto, one or more heteroatoms.

Also, unless expressly stated, as used herein, "substituted" in the term "substituted or unsubstituted" means substituted with one or more substituents selected from the group consisting of deuterium, halogen, an amino group, a nitrile group, a nitro group, a C₁-C₂₀ alkyl group, a C₁-C₂₀ alkoxyl group, a C₁-C₂₀ alkylamine group, a C₁-C₂₀ alkylthiopen group, a C₆-C₂₀ arylthiopen group, a C₂-C₂₀ alkenyl group, a C₂-C₂₀ alkynyl group, a C₃-C₂₀ cycloalkyl group, a C₆-C₂₀ aryl group, a C₆-C₂₀ aryl group substituted by deuterium, a C₈-C₂₀ arylalkenyl group, a silane group, a boron group, a germanium group, and a C₂-C₂₀ heterocyclic group, but is not limited to these substituents.

In this specification, the 'group name' corresponding to the aryl group, arylene group, heterocyclic group, etc., as examples of each symbol and its substituent, may be written as the 'name of the group reflecting the valence', but is written as the 'parent compound name'. For example, in the case of 'phenanthrene', a type of aryl group, the name of the group may be written by distinguishing the valence, such as the monovalent 'group' is 'phenanthryl' and the divalent group is 'phenanthrylene', but may be written as 'phenanthrene', which is the name of the parent compound, regardless of the valence. Similarly, in the case of pyrimidine, it can be written as 'pyrimidine' regardless of the valence, or it can be written as the 'name of the group' of the valence, such as pyrimidineyl group in the case of monovalent group, pyrimidineylene in the case of divalent group, etc. Additionally, in this specification, when describing compound names or substituent names, numbers or alphabets indicating positions may be omitted. For example, pyrido[4,3-d]pyrimidine to pyridopyrimidine, benzofuro[2,3-d]pyrimidine to benzofuropyrimidine, 9,9-dimethyl-9H-fluorene can be described as dimethylfluorene, etc. Therefore, both benzo[g]quinoxaline and benzo[f]quinoxaline can be described as benzoquinoxaline.

Also, unless there is an explicit explanation, the formula used in the present invention is the same as the definition of the substituent by the exponent definition of the following formula.

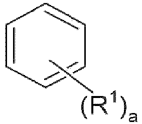

Here, when a is an integer of 0, the substituent R¹ is absent, when a is an integer of 1, the sole substituent R¹ is linked to any one of the carbon constituting the benzene ring, when a is an integer of 2 or 3, each is combined as follows, where R¹ may be the same or different from each other, when a is an integer of 4 to 6, it is bonded to the carbon of the benzene ring in a similar manner, while the indication of the hydrogen bonded to the carbon forming the benzene ring is omitted.

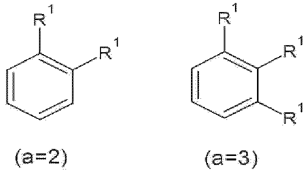

Unless otherwise expressly stated, the terms "ortho", "meta", and "para" used in the present invention refer to the substitution positions of all substituents, and the ortho position refers to a compound in which the position of the substituent is immediately adjacent, for example, when benzene is used, it means 1 or 2 position, and the meta position is the next substitution position of the neighbor substitution position, when benzene as an example stands for 1 or 3 position, and the para position is the next substitution position of the meta position, which means 1 and 4 position when benzene is taken as an example. A more detailed example of the substitution position is as follows, and it can be confirmed that the ortho-, and meta- position are substituted by non-linear type and para- positions are substituted by linear type.

### [Example of ortho-position]

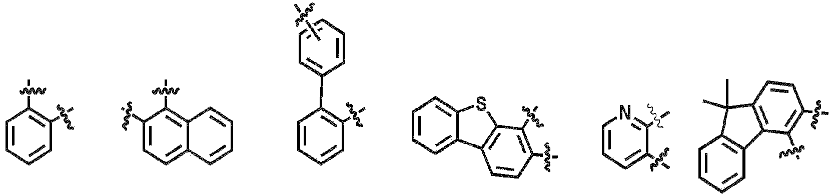

### [Example of meta-position]

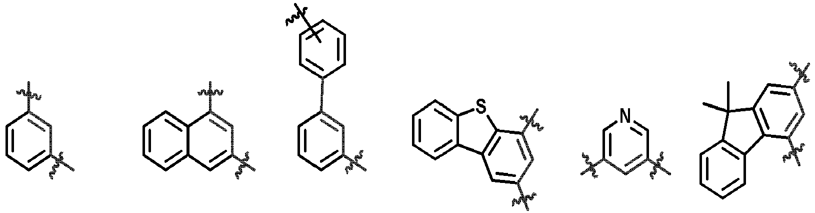

### [Example of para-position]

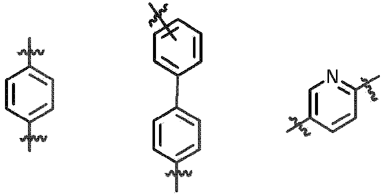

Hereinafter, a compound according to one aspect of the present invention and an organic electronic element comprising the same will be described.

The present invention provides a compound represented by Formula 1. 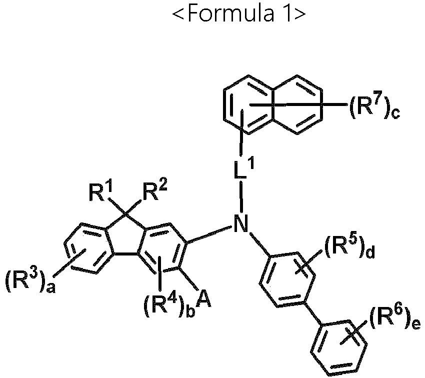 Wherein:
R¹ and R² are the same or different from each other and are independently C₁-C₅₀ alkyl groups substituted or unsubstituted with deuterium; more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.
R³, R⁴, R⁵, R⁶ and R⁷ are the same or different from each other and are independently selected from the group consisting of a hydrogen; deuterium; a C₆-C₆₀ aryl group; a fluorenylene group; a C₂-C₆₀ heterocyclic group including at least one heteroatom of O, N, S, Si or P; and a fused ring group of a C₃-C₆₀ aliphatic ring and a C₆-C₆₀ aromatic ring; a C₁-C₅₀ alkyl group; a C₂-C₂₀ alkenyl group; a C₂-C₂₀ alkynyl group; a C₁-C₃₀ alkoxyl group; and a C₆-C₃₀ aryloxy group; and a C₃-C₆₀ aliphatic ring; or may be bonded to adjacent groups to form an aromatic ring or a heteroaromatic ring,
Wherein R³, R⁴, R⁵, R⁶ and R⁷ are an aryl group, preferably an C₆-C₃₀ aryl group, more preferably an C₆-C₂₅ aryl group, an C₆-C₁₈ aryl group or an C₆-C₁₂ aryl group, such as phenyl, biphenyl, terphenyl, naphthalene, phenanthrene, chrysene, etc.

Wherein R³, R⁴, R⁵, R⁶ and R⁷ are a heterocyclic group, preferably a C₂-C₃₀ heterocyclic group, more preferably a C₂-C₂₅ heterocyclic group, a C₂-C₁₈ heterocyclic group, or a C₂-C₁₂ heterocyclic group, such as pyrazine, thiophene, pyridine, pyrimidine, quinoline, pyrimidoindole, 5-phenyl-5H-pyrimido[5,4-b]indole, quinazoline, quinoxaline, benzoquinazoline, carbazole, dibenzoquinazoline, benzofuran, benzothiophene, dibenzofuran, dibenzothiophene, benzothienopyrimidine, benzofuropyrimidine, phenothiazine, phenylphenothiazine, naphthobenzofuran, naphthobenzothiophene, etc.

Wherein R³, R⁴, R⁵, R⁶ and R⁷ are a fused ring group, preferably a fused ring group of an C₃-C₃₀ aliphatic ring and an C₆-C₃₀ aromatic ring, more preferably a fused ring group of an C₃-C₂₅ aliphatic ring and an C₆-C₂₅ aromatic ring.

Wherein R³, R⁴, R⁵, R⁶ and R⁷ are an alkyl group, preferably a C₁-C₃₀ alkyl group, more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.

Wherein R³, R⁴, R⁵, R⁶ and R⁷ are an alkoxyl group, preferably a C₁-C₂₅ alkoxyl group, a C₁-C₁₈ alkoxyl group or a C₁-C₁₂ alkoxyl group.

Wherein R³, R⁴, R⁵, R⁶ and R⁷ are an aryloxy group, preferably a C₆-C₂₅ aryloxy group, a C₆-C₁₈ an aryloxy group or a C₆-C₁₂ aryloxy group.

Wherein R³, R⁴, R⁵, R⁶ and R⁷ are an aliphatic ring group, preferably a C₃-C₃₀ aliphatic ring group, more preferably a C₃-C₂₅ aliphatic ring group, a C₃-C₁₈ aliphatic ring group, and a C₃-C₁₂ aliphatic ring group, and specifically, cyclobutane, cyclopentane, cyclohexane, bicycloheptane, adamantyl, etc.

A is an a C₁-C₅₀ alkyl group; more preferably a C₁-C₂₅ alkyl group, a C₁-C₁₈ alkyl group or a C₁-C₁₂ alkyl group, such as a methyl group, ethyl group, propyl group, isopropyl group, butyl group, t-butyl group, pentyl group, etc.

L¹ is selected from the group consisting of a C₆-C₆₀ arylene group; a fluorenylene group; a C₂-C₆₀ heteroarylene group including at least one heteroatom of O, N, S, Si or P; a C₃-C₆₀ aliphatic ring; and a fused ring group of a C₃-C₆₀ aliphatic ring and a C₆-C₆₀ aromatic ring;

Wherein L¹ is an arylene group, preferably an C₆-C₃₀ arylene group, more preferably an C₆-C₂₅ arylene group, an C₆-C₁₈ arylene group or an C₆-C₁₂ arylene group, such as phenylene, biphenylene, naphthylene, terphenylene, anthracenylene, etc.

Wherein L¹ is an heteroarylene group, preferably an C₂-C₃₀ heteroarylene group, more preferably an C₂-C₂₅ heteroarylene group, an C₂-C₁₈ heteroarylene group or an C₂-C₁₂ heteroarylene group

Wherein L¹ is an aliphatic ring group, preferably a C₃-C₃₀ aliphatic ring group, more preferably a C₃-C₂₅ aliphatic ring group, a C₃-C₁₈ aliphatic ring group, and a C₃-C₁₂ aliphatic ring group, and specifically, cyclobutane, cyclopentane, cyclohexane, bicycloheptane, adamantyl, etc.

Wherein L¹ is a fused ring group, preferably a fused ring group of a C₃-C₃₀ aliphatic ring and an C₆-C₃₀ aromatic ring, more preferably a fused ring group of an C₃-C₂₅ aliphatic ring and an C₆-C₂₅ aromatic ring.
a and d are independently integers from 0 to 4, b is an integer from 0 to 2, c is an integer from 0 to 7, and e is an integer from 0 to 5,
wherein the aryl group, arylene group, heteroarylene group, heterocyclic group, fluorenyl group, fluorenylene group, aliphatic ring group, fused ring group, alkyl group, alkenyl group, alkynyl group, alkoxyl group and aryloxy group may be substituted with one or more substituents selected from the group consisting of deuterium; halogen; silane group; siloxane group; boron group; germanium group; cyano group; nitro group; C₁-C₂₀ alkylthio group; C₁-C₂₀ alkoxyl group; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group; a C₃-C₂₀ aliphatic ring; a C₇-C₂₀ arylalkyl group; a C₈-C₂₀ arylalkenyl group; and a C₇-C₂₀ alkylaryl group; also the hydrogen of these substituents may be further substituted with one or more deuterium, and also the substituents may be bonded to each other to form a saturated or unsaturated ring, wherein the term 'ring' means a C₃-C₆₀ aliphatic ring or a C₆-C₆₀ aromatic ring or a C₂-C₆₀ heterocyclic group or a fused ring formed by the combination thereof.

Also, Formula 1 may be represented by Formulas 1-1 or 1-2.

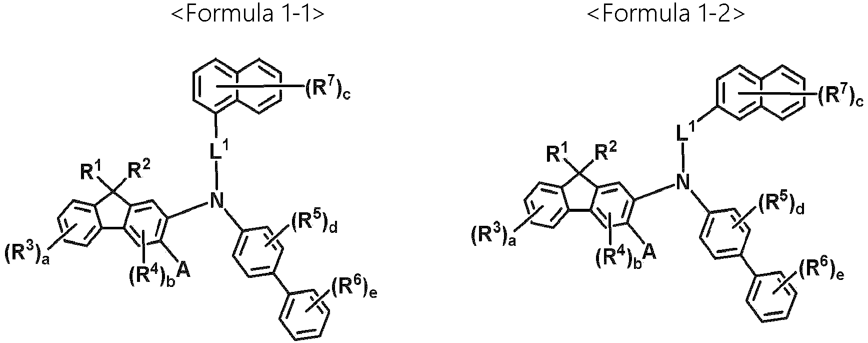

Wherein, R¹, R², R³, R⁴, R⁵, R⁶, R⁷, L¹, A, a, b, c, d and e are the same as defined in Formula 1.

Also, A is represented by any one of the following Formulas A-1 to A-3.

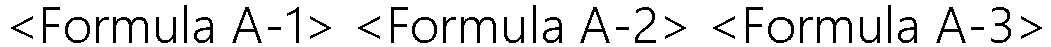

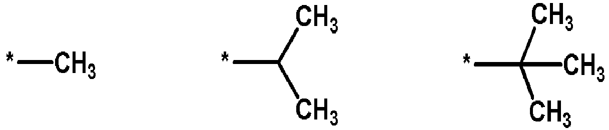

Wherein, hydrogen can be further substituted with one or more deuterium.

Also, L¹ is represented by any one of the following Formulas L-1 to L-6: 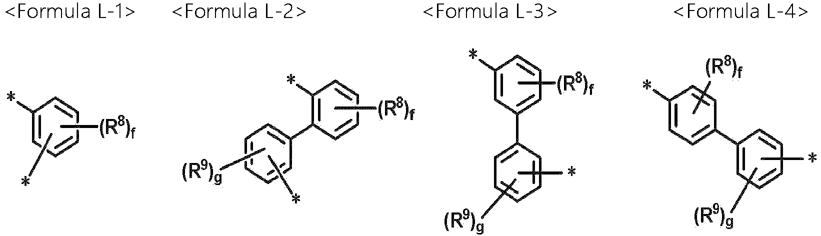 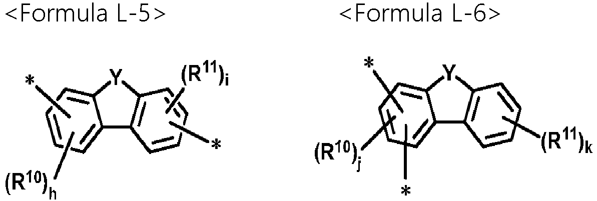 Wherein:
R⁸, R⁹, R¹⁰ and R¹¹ are each the same or different, and each independently selected from the group consisting of a hydrogen; deuterium; halogen; cyano group; nitro group; C₁-C₂₀ alkoxyl group; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group including at least one heteroatom of O, N, S, Si or P; C₃-C₂₀ aliphatic group; C₇-C₂₀ arylalkyl group; C₈-C₂₀ arylalkenyl group; and a C₇-C₂₀ alkylaryl group;
or an adjacent plurality of R⁸s or plurality of R⁹s or plurality of R¹⁰s or plurality of R¹¹s may be bonded to each other to form a ring,
Y is O, S, NR or CR'R",
Wherein R, R', R" are each independently selected from the group consisting of an C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group including at least one heteroatom of O, N, S, Si or P; C₃-C₂₀ aliphatic group; and a fused ring group of a C₃-C₂₀ aliphatic ring and a C₆-C₂₀ aromatic ring;
f, g and k are independently integers from 0 to 4, h and i are independently integers from 0 to 3, j is an integer from 0 to 2,
* indicates the position to be bonded.

Specifically, the compound represented by Formula 1 may be any one of the following compounds P-1 to P-80, but is not limited thereto.

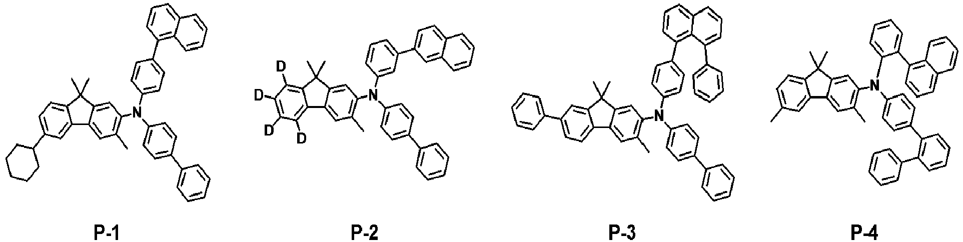

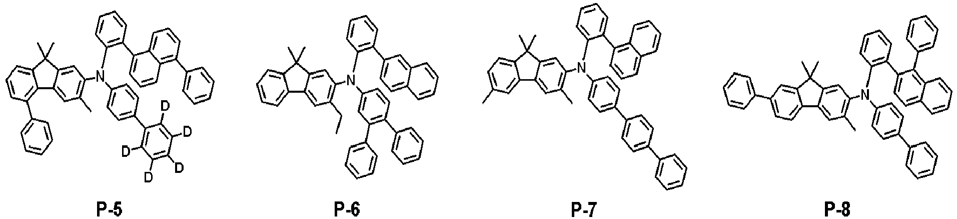

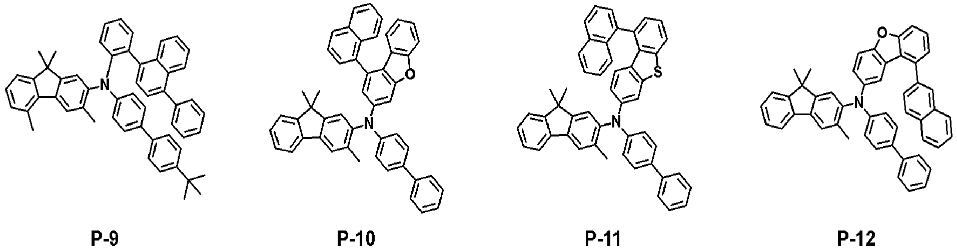

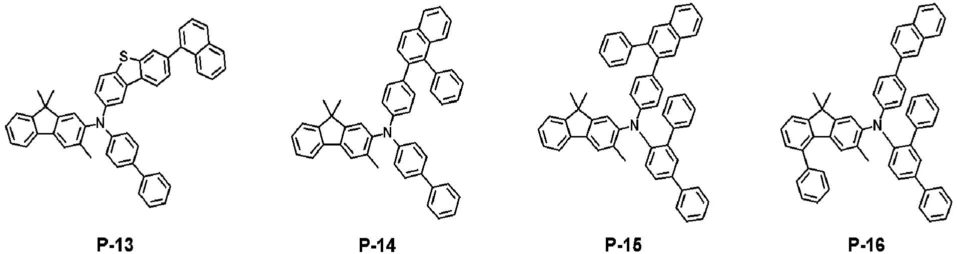

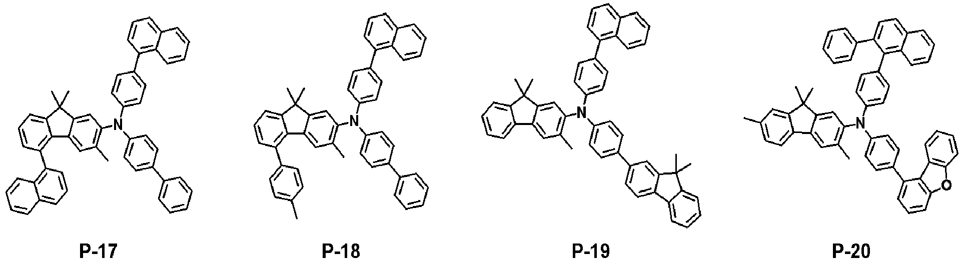

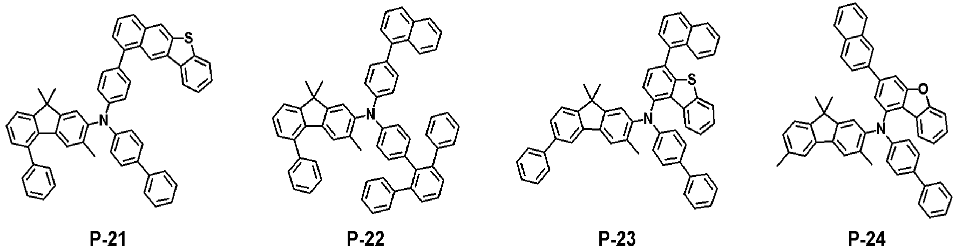

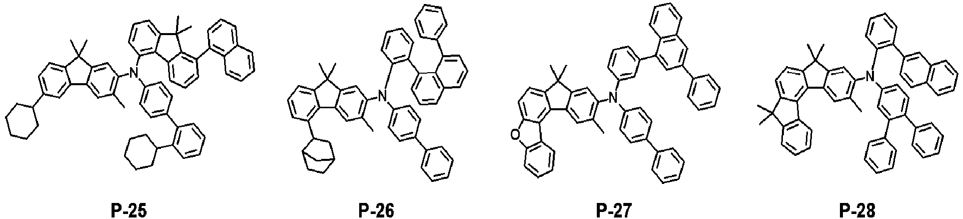

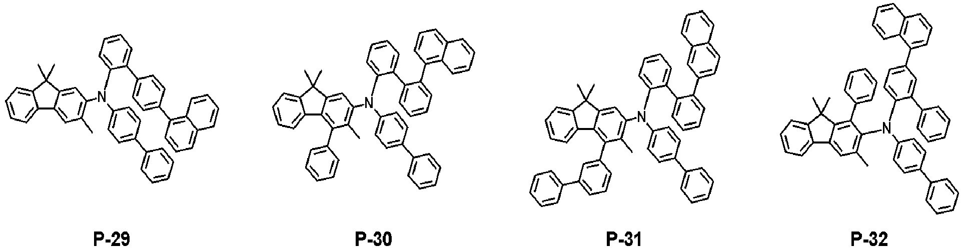

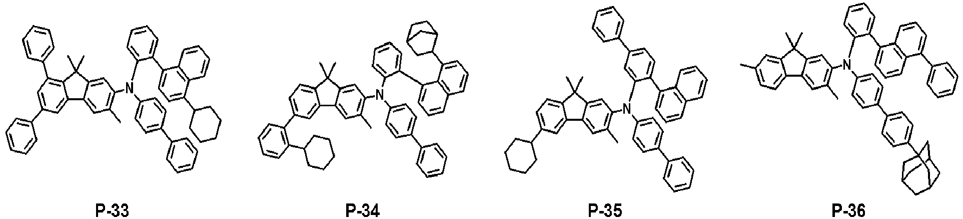

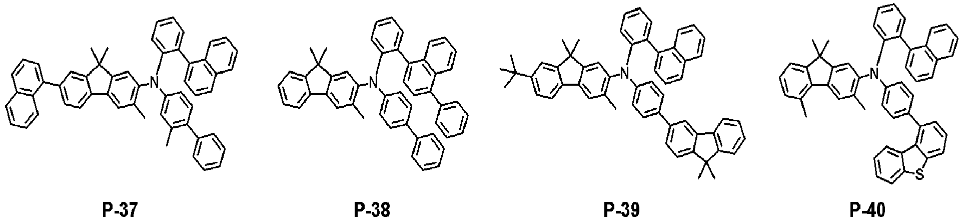

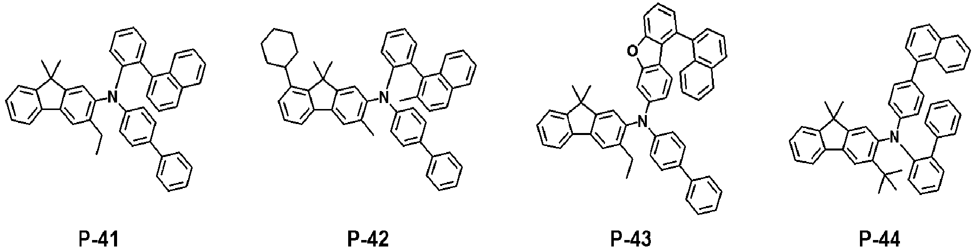

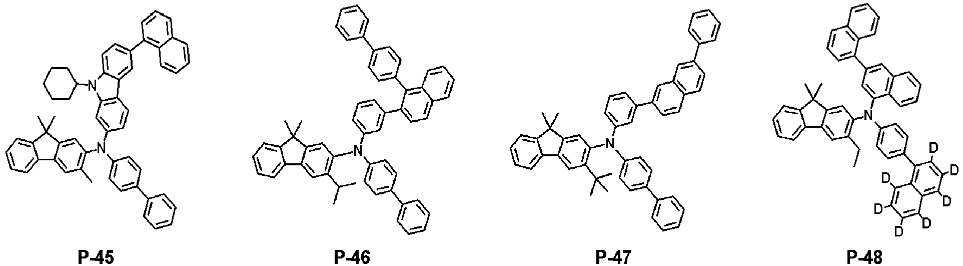

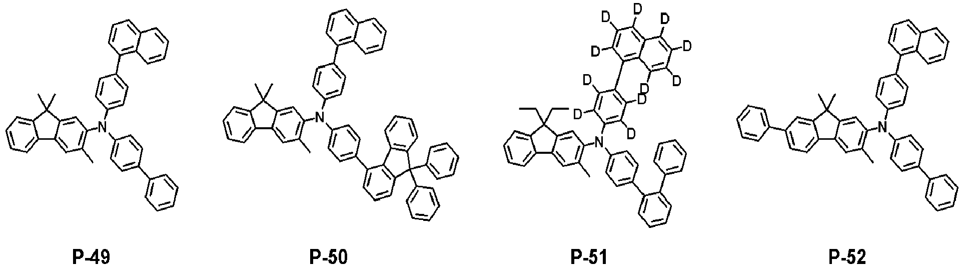

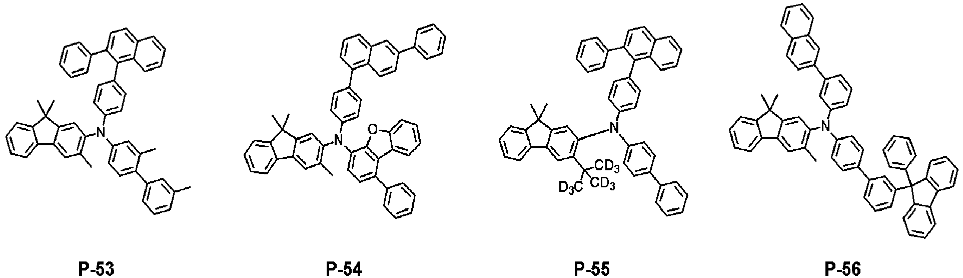

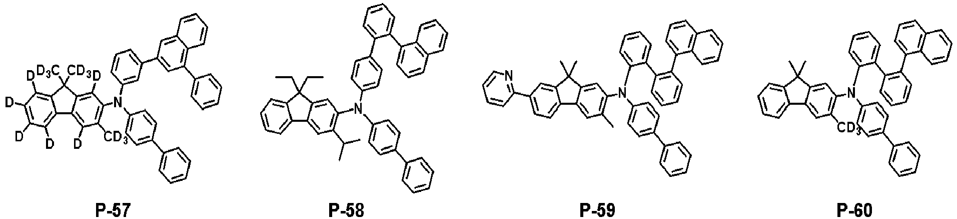

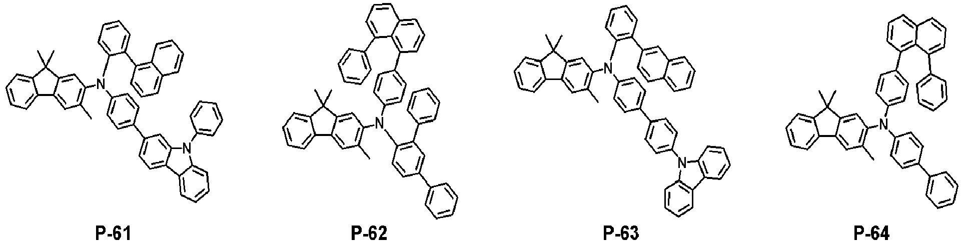

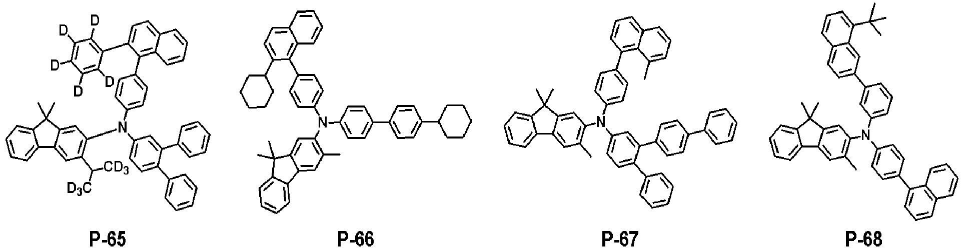

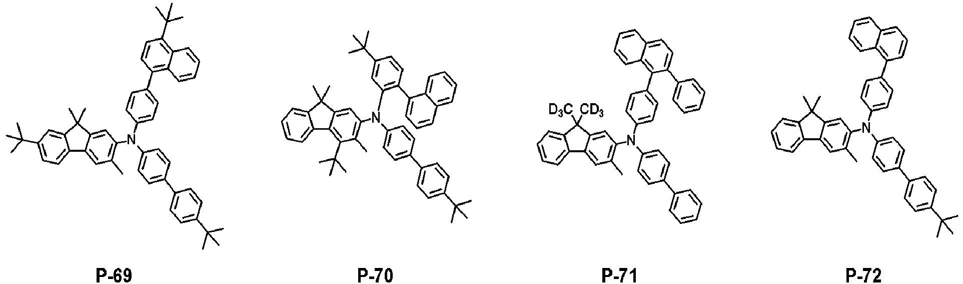

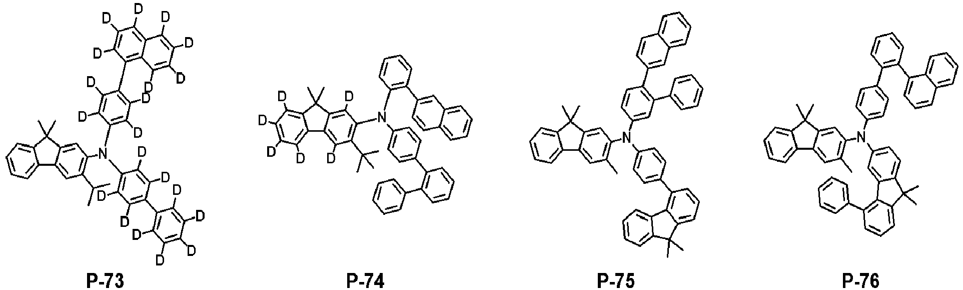

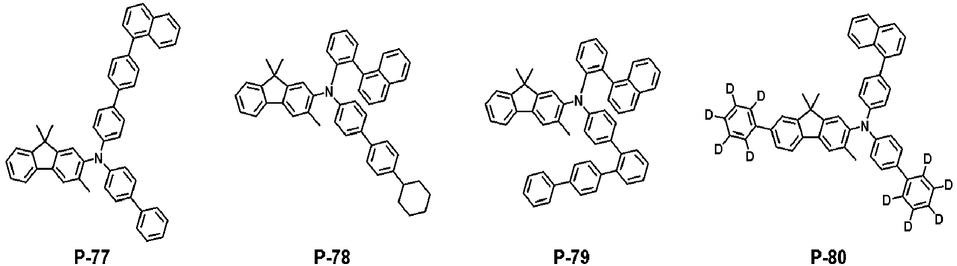

In another aspect, the present invention provides a method for reusing a compound of Formula 1 comprising:
recovering a crude organic light emitting material comprising the compound of Formula 1 from a deposition apparatus used in the process for depositing the organic emitting material to prepare an organic light emitting device;
removing impurities from the crude organic light emitting material;
recovering the organic light emitting material after the impurities are removed; and purifying the recovered organic light emitting material to have a purity of 99.9% or higher.

The step of removing impurities from the crude organic light emitting material recovered from the deposition apparatus may preferably comprise performing a pre-purification process to obtain a purity of 98% or more by recrystallization in a recrystallization solvent.

The recrystallization solvent may be preferably a polar solvent having a polarity index (PI) of 5.5 to 7.2.

The recrystallization solvent may preferably be used by mixing a polar solvent having a polarity value of 5.5 to 7.2 and a non-polar solvent having a polarity value of 2.0 to 4.7.

When a mixture of a polar solvent and a non-polar solvent is used, the recrystallization solvent may be used in an amount of 15% (v/v) or less of the non-polar solvent compared to the polar solvent.

The recrystallization solvent is preferably a single solvent of N-Methylpyrrolidone (NMP); or a polar solvent mixed any one selected from the group consisting of 1,3-Dimethyl-2-imidazolidinone, 2-pyrrolidone, N,N-Dimethyl formamide, Dimethyl acetamide, and Dimethyl sulfoxide to the N-Methylpyrrolidone; or alone; or mixed non-polar solvents; selected from the group consisting of Toluene, Dichloromethane (DCM), Dichloroethane (DCE), Tetrahydrofuran (THF), Chloroform, Ethyl acetate and Butanone; or a mixture of a polar solvent and a non-polar solvent.

The pre-purification process may comprise a step of precipitating crystals of by cooling to 0° C. to 5° C. after dissolving the crude organic light emitting material recovered from the deposition apparatus in a polar solvent at 90° C. to 120° C.

The pre-purification process may comprise a step of precipitating crystals by cooling to 35°C to 40°C, adding a non-polar solvent, and then cooling to 0°C to 5°C. after dissolving the crude organic light emitting material recovered from the deposition apparatus in a polar solvent at 90° C. to 120° C.

The pre-purification process may comprise a step of precipitating crystals while concentrating the solvent and removing the non-polar solvent, after dissolving the crude organic light emitting material recovered from the deposition apparatus in a non-polar solvent.

The pre-purification process may comprise a step of recrystallizing again with a non-polar solvent after recrystallizing first with a polar solvent.

The step of purifying the recovered impurities to a purity of 99.9% or higher may comprise performing an adsorption separation process to adsorb and remove impurities by adsorbing on the adsorbent.

The adsorbent may be activated carbon, silica gel, alumina, or a material for known adsorption purposes.

The step of purifying the recovered impurities to a purity of 99.9% or higher may comprise performing sublimation purification.

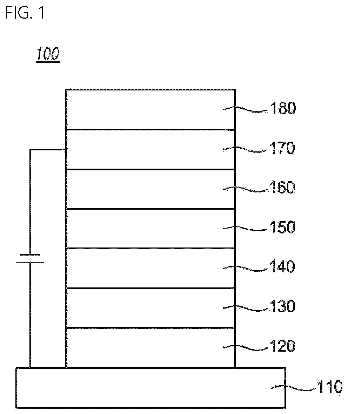

Referring to FIG. 1, the organic electronic element (100) according to the present invention comprises a first electrode (110), a second electrode (170), an organic material layer comprising single compound or 2 or more compounds represented by Formula 1 between the first electrode (110) and the second electrode (170). Wherein, the first electrode (110) may be an anode or a positive electrode, and the second electrode (170) may be a cathode or a negative electrode. In the case of an inverted organic electronic element, the first electrode may be a cathode, and the second electrode may be an anode.

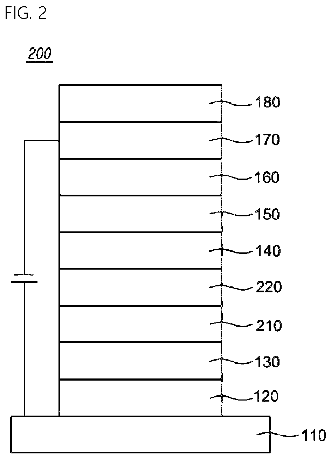

The organic material layer may sequentially comprise a hole injection layer (120), a hole transport layer (130), an emitting layer (140), an electron transport layer(150), and an electron injection layer (160) on the first electrode(110). Here, the remaining layers except the emitting layer (140) may not be formed. The organic material layer may further comprise a hole blocking layer, an electron blocking layer, an emitting-auxiliary layer (220), a buffer layer (210), etc., and the electron transport layer (150), etc. may serve as a hole blocking layer (see FIG. 2).

Also, the organic electronic element according to an embodiment of the present invention may further include a protective layer or a light efficiency enhancing layer (180). Wherein the light efficiency enhancing layer is formed on one of both surfaces of the first electrode that is not in contact with the organic material layer or on one of both surfaces of the second electrode that is not in contact with the organic material layer. The compound according to an embodiment of the present invention applied to the organic material layer may be used as a material for a hole injection layer (120), a hole transport layer (130), an emitting-auxiliary layer (220), an electron transport au xiliary layer, an electron transport layer (150), an electron injection layer (160), a host or dopant of an emitting layer (140), or the light efficiency enhancing layer. Preferably, for example, a compound according to Formula 1 of the present invention can be used as a material of a hole transport layer.

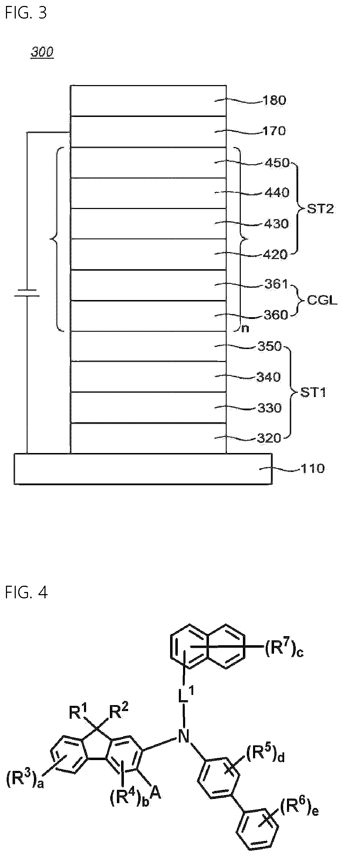

The organic material layer may comprise 2 or more stacks comprising a hole transport layer, an emitting layer and an electron transport layer sequentially formed on the anode, and may further comprise a charge generation layer formed between the 2 or more stacks (see FIG. 3).

Otherwise, even if the same core is used, the band gap, the electrical characteristics, the interface characteristics, etc. may vary depending on which substituent is bonded at which position, therefore the choice of core and the combination of sub-substituents associated therewith is also very important, and in particular, when the optimal combination of energy levels and T1 values, and unique properties of materials(mobility, interfacial characteristics, etc.) of each organic material layer is achieved, a long life span and high efficiency can be achieved at the same time.

The organic electroluminescent device according to an embodiment of the present invention may be manufactured using a PVD (physical vapor deposition) method. For example, a metal or a metal oxide having conductivity or an alloy thereof is deposited on a substrate to form a cathode, and the organic material layer including the hole injection layer(120), the hole transport layer(130), the emitting layer(140), the electron transport layer(150), and the electron injection layer(160) is formed thereon, and then a material that can be used as a cathode is deposited thereon.

Also, the present invention provides the organic electronic element wherein the organic material layer is formed by one of a spin coating process, a nozzle printing process, an inkjet printing process, a slot coating process, a dip coating process or a roll-to-roll process, and the organic material layer comprises the compound as an electron transport material.

As another specific example, a compound of the same or different types represented by Formula 1 is mixed and used in the organic material layer.

Additionally, the present invention provides a hole transport layer composition comprising a compound represented by Formula 1, and provides an organic electronic element comprising the hole transport layer.

Also, the present invention also provides an electronic device comprising a display device comprising the organic electronic element; and a control unit for driving the display device.

According to another aspect, the present invention provides a display device wherein the organic electronic element is at least one of an OLED, an organic solar cell, an organic photo conductor, an organic transistor (organic TFT) and an element for monochromic or white illumination. Here, the electronic device may be a wired/wireless communication terminal which is currently used or will be used in the future, and covers all kinds of electronic devices including a mobile communication terminal such as a cellular phone, a personal digital assistant(PDA), an electronic dictionary, a point-to-multipoint(PMP), a remote controller, a navigation unit, a game player, various kinds of TVs, and various kinds of computers.

Hereinafter, Synthesis examples of the compound represented by Formula 1 of the present invention, and preparation examples of the organic electronic element of the present invention will be described in detail by way of example, but are not limited to the following examples.

### [Synthesis Example 1]

The compound (final products) represented by Formula 1 according to the present invention can be synthesized by a reaction as in Reaction Scheme 1, but is not limited thereto. (Hal¹= Cl, I or Cl)

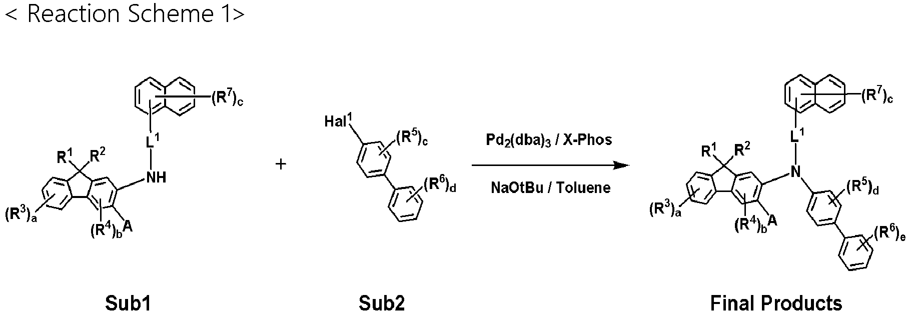

Meanwhile, compounds belonging to Sub 1 may be, but are not limited to, the compounds below, and Table 1 shows the FD-MS (Field Desorption-Mass Spectrometry) values of compounds belonging to Sub 1.

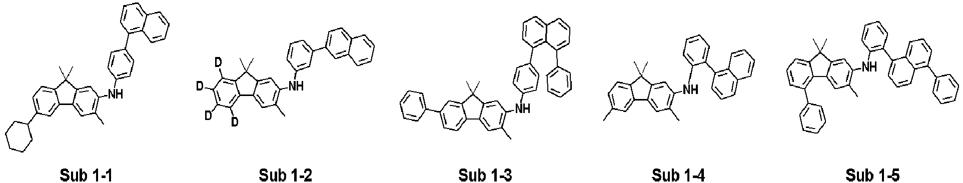

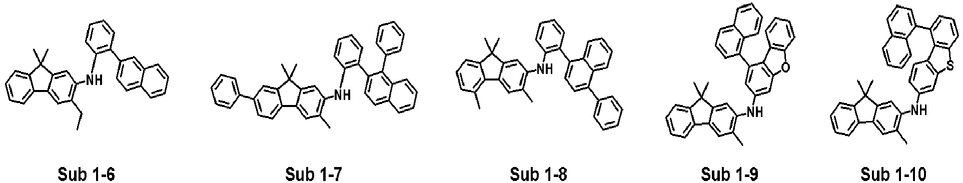

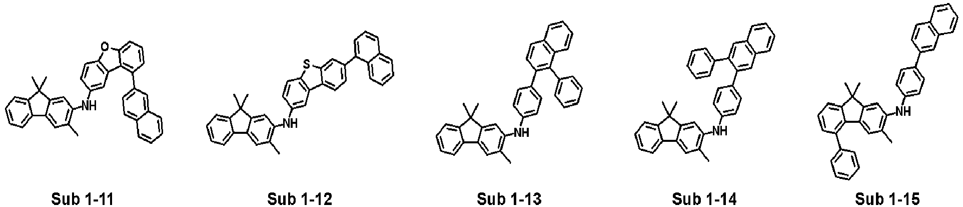

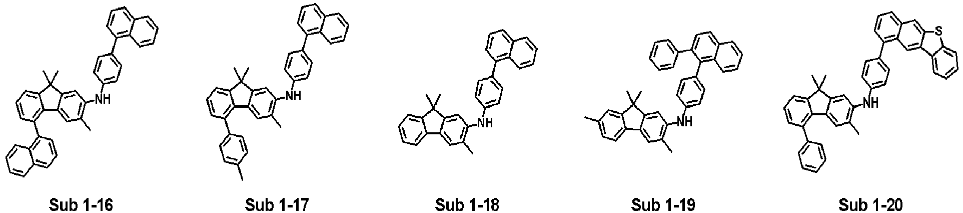

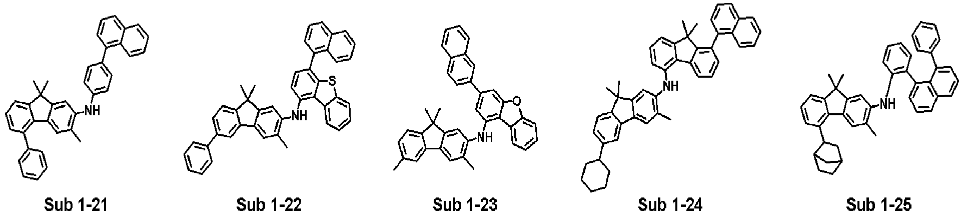

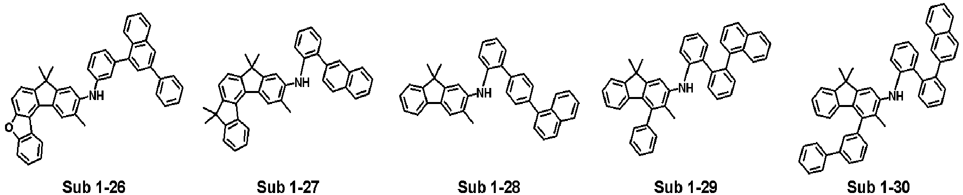

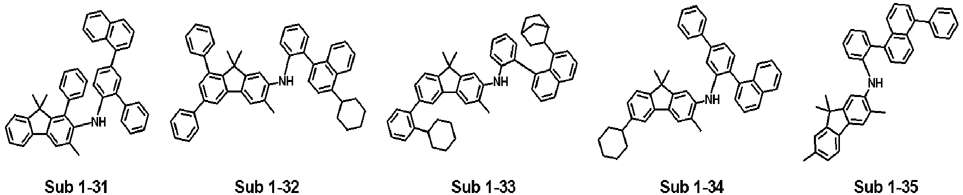

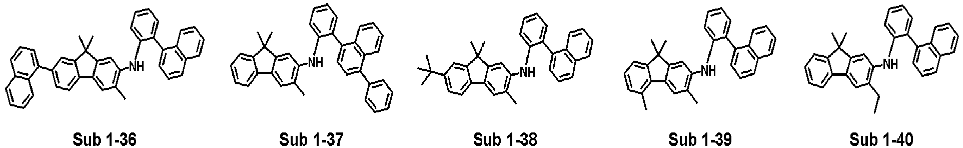

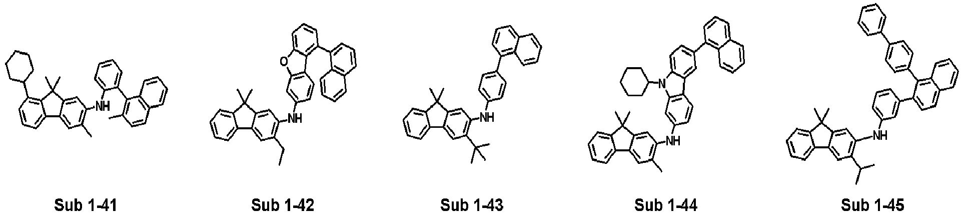

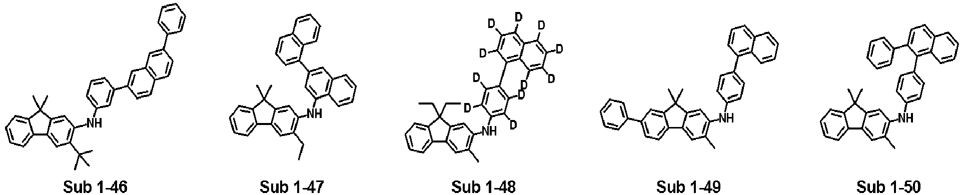

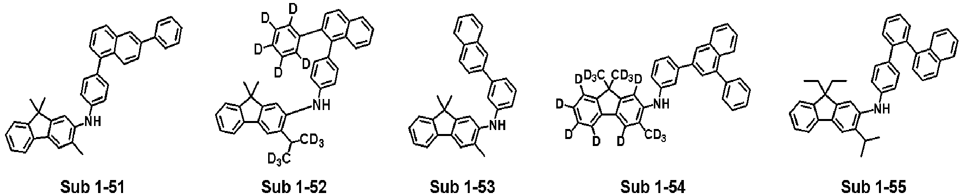

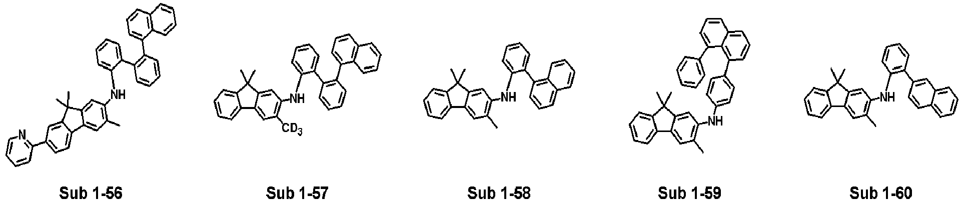

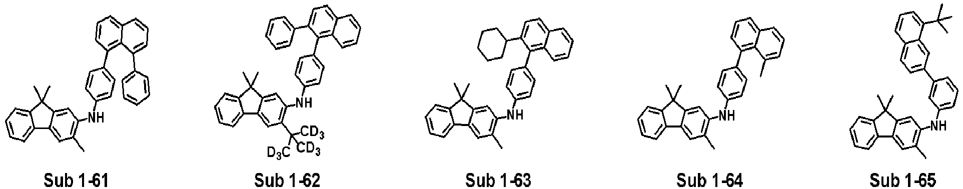

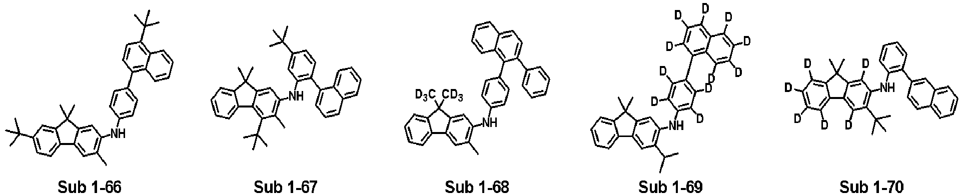

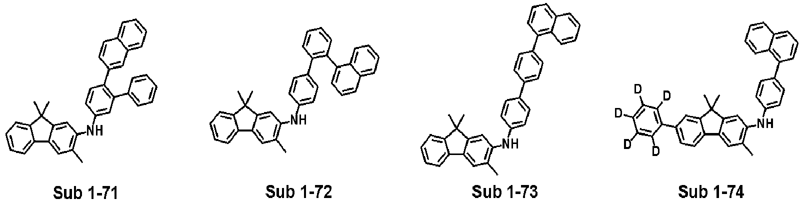

**[Table 1]**

| compound | FD-MS | compound | FD-MS |
|---|---|---|---|
| Sub 1-1 | m/z=507.29 (C₃₈H₃₇N=507.72) | Sub 1-2 | m/z=429.24 (C₃₂H₂₃D₄N=429.60) |
| Sub 1-3 | m/z=577.28 (C₄₄H₃₅N=577.77) | Sub 1-4 | m/z=439.23 (C₃₃H₂₉N=439.60) |
| Sub 1-5 | m/z=577.28 (C₄₄H₃₅N=577.77) | Sub 1-6 | m/z=439.23 (C₃₃H₂₉N=439.60) |
| Sub 1-7 | m/z=577.28 (C₄₄H₃₅N=577.77) | Sub 1-8 | m/z=515.26 (C₃₉H₃₃N=515.70) |
| Sub 1-9 | m/z=515.22 (C₃₈H₂₉NO=515.66) | Sub 1-10 | m/z=531.20 (C₃₈H₂₉NS=531.72) |
| Sub 1-11 | m/z=515.22 (C₃₈H₂₉NO=515.66) | Sub 1-12 | m/z=531.20 (C₃₈H₂₉NS=531.72) |
| Sub 1-13 | m/z=501.25 (C₃₈H₃₁N=501.67) | Sub 1-14 | m/z=501.25 (C₃₈H₃₁N=501.67) |
| Sub 1-15 | m/z=501.25 (C₃₈H₃₁N=501.67) | Sub 1-16 | m/z=551.26 (C₄₂H₃₃N=551.73) |
| Sub 1-17 | m/z=515.26 (C₃₉H₃₃N=515.70) | Sub 1-18 | m/z=425.21 (C₃₂H₂₇N=425.58) |
| Sub 1-19 | m/z=515.26 (C₃₉H₃₃N=515.70) | Sub 1-20 | m/z=607.23 (C₄₄H₃₃NS=607.81) |
| Sub 1-21 | m/z=501.25 (C₃₈H₃₁N=501.67) | Sub 1-22 | m/z=607.23 (C₄₄H₃₃NS=607.81) |
| Sub 1-23 | m/z=529.24 (C₃₉H₃₁N=529.68) | Sub 1-24 | m/z=623.36 (C₄₇H₄₅N=623.88) |
| Sub 1-25 | m/z=595.32 (C₄₅H₄₁N=595.83) | Sub 1-26 | m/z=591.26 (C₄₄H₃₃NO=591.75) |
| Sub 1-27 | m/z=541.28 (C₄₁H₃₅N=541.74) | Sub 1-28 | m/z=501.25 (C₃₈H₃₁N=501.67) |
| Sub 1-29 | m/z=577.28 (C₄₄H₃₅N=577.77) | Sub 1-30 | m/z=653.31 (C₅₀H₃₉N=653.87) |
| Sub 1-31 | m/z=577.28 (C₄₄H₃₅N=577.77) | Sub 1-32 | m/z=659.36 (C₅₀H₄₅N=659.92) |
| Sub 1-33 | m/z=677.40 (C₅₁H₅₁N=677.98) | Sub 1-34 | m/z=583.32 (C₄₄H₄₁N=583.82) |
| Sub 1-35 | m/z=515.26 (C₃₉H₃₃N=515.70) | Sub 1-36 | m/z=551.26 (C₄₂H₃₃N=551.73) |
| Sub 1-37 | m/z=501.25 (C₃₈H₃₁N=501.67) | Sub 1-38 | m/z=481.28 (C₃₆H₃₅N=481.68) |
| Sub 1-39 | m/z=439.23 (C₃₃H₂₉N=439.60) | Sub 1-40 | m/z=439.23 (C₃₃H₂₉N=439.60) |
| Sub 1-41 | m/z=521.31 (C₃₉H₃₉N=521.75) | Sub 1-42 | m/z=529.24 (C₃₉H₃₁N=529.68) |
| Sub 1-43 | m/z=467.26 (C₃₅H₃₃N=467.66) | Sub 1-44 | m/z=596.32 (C₄₄H₄₀N₂=596.82) |
| Sub 1-45 | m/z=605.31 (C₄₆H₃₉N=605.82) | Sub 1-46 | m/z=543.29 (C₄₁H₃₇N=543.75) |
| Sub 1-47 | m/z=489.25 (C₃₇H₃₁N=489.66) | Sub 1-48 | m/z=464.31 (C₃₄H₂₀D₁₁N=464.70) |
| Sub 1-49 | m/z=501.25 (C₃₈H₃₁N=501.67) | Sub 1-50 | m/z=501.25 (C₃₈H₃₁N=501.67) |
| Sub 1-51 | m/z=501.25 (C₃₈H₃₁N=501.67) | Sub 1-52 | m/z=540.35 (C₄₀H₂₄D₁₁N=540.79) |
| Sub 1-53 | m/z=425.21 (C₃₂H₂₇N=425.58) | Sub 1-54 | m/z=516.34 (C₃₈H₁₆D₁₅N=516.76) |
| Sub 1-55 | m/z=557.31 (C₄₂H₃₉N=557.78) | Sub 1-56 | m/z=578.27 (C₄₃H₃₄N₂=578.76) |
| Sub 1-57 | m/z=504.26 (C₃₈H₂₈D₃N=504.69) | Sub 1-58 | m/z=425.21 (C₃₂H₂₇N=425.58) |
| Sub 1-59 | m/z=501.25 (C₃₈H₃₁N=501.67) | Sub 1-60 | m/z=425.21 (C₃₂H₂₇N=425.58) |
| Sub 1-61 | m/z=501.25 (C₃₈H₃₁N=501.67) | Sub 1-62 | m/z=552.35 (C₄₁H₂₈D₉N=552.81) |
| Sub 1-63 | m/z=507.29 (C₃₈H₃₇N=507.72) | Sub 1-64 | m/z=439.23 (C₃₃H₂₉N=439.60) |
| Sub 1-65 | m/z=481.28 (C₃₆H₃₅N=481.68) | Sub 1-66 | m/z=537.34 (C₄₀H₄₃N=537.79) |
| Sub 1-67 | m/z=537.34 (C₄₀H₄₃N=537.79) | Sub 1-68 | m/z=507.28 (C₃₈H₂₅D₆N=507.71) |
| Sub 1-69 | m/z=464.31 (C₃₄H₂₀D₁₁N=464.70) | Sub 1-70 | m/z=473.30 (C₃₅H₂₇D₆N=473.69) |
| Sub 1-71 | m/z=501.25 (C₃₈H₃₁N=501.67) | Sub 1-72 | m/z=501.25 (C₃₈H₃₁N=501.67) |
| Sub 1-73 | m/z=501.25 (C₃₈H₃₁N=501.67) | Sub 1-74 | m/z=506.28 (C₃₈H₂₆D₅N=506.70) |

Compounds belonging to Sub 2 may include, but are not limited to, the compounds below, and Table 2 shows the FD-MS (Field Desorption-Mass Spectrometry) values of compounds belonging to Sub 2.

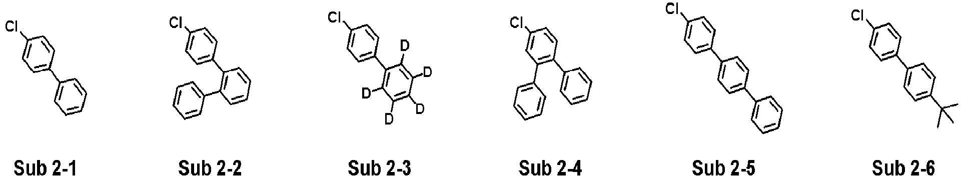

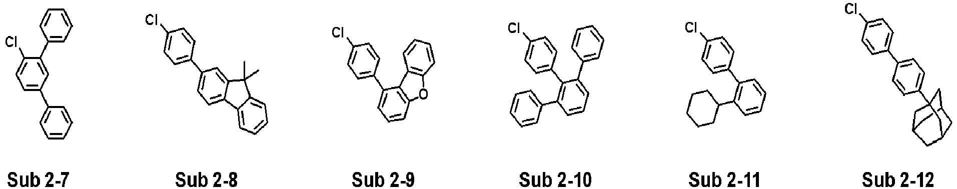

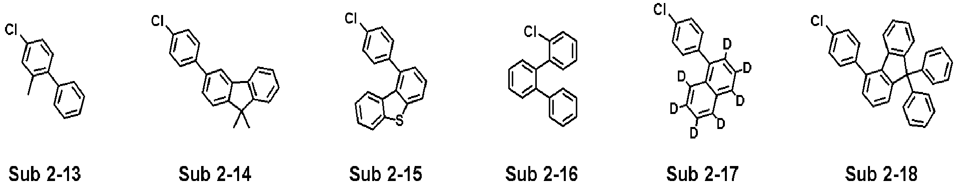

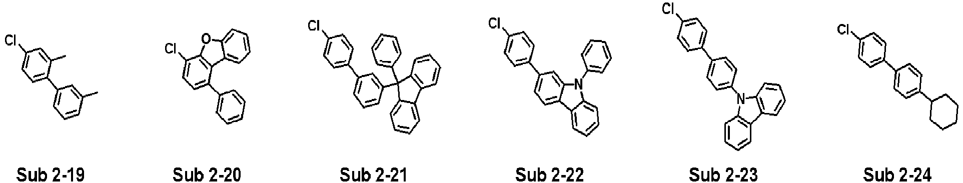

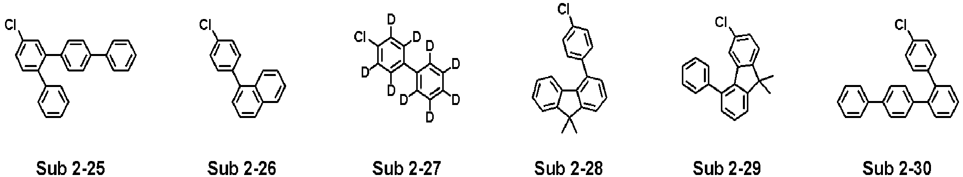

**[Table 2]**

| compound | FD-MS | compound | FD-MS |
|---|---|---|---|
| Sub 2-1 | m/z=188.04 (C₁₂H₉Cl=188.65) | Sub 2-2 | m/z=264.07 (C₁₈H₁₃Cl=264.75) |
| Sub 2-3 | m/z=193.07 (C₁₂H₄D₅Cl=193.68) | Sub 2-4 | m/z=264.07 (C₁₈H₁₃Cl=264.75) |
| Sub 2-5 | m/z=264.07 (C₁₈H₁₃Cl=264.75) | Sub 2-6 | m/z=244.10 (C₁₆H₁₇Cl=244.76) |
| Sub 2-7 | m/z=264.07 (C₁₈H₁₃Cl=264.75) | Sub 2-8 | m/z=304.10 (C₂₁H₁₇Cl=304.82) |
| Sub 2-9 | m/z=278.05 (C₁₈H₁₁ClO=278.74) | Sub 2-10 | m/z=340.10 (C₂₄H₁₇Cl=340.85) |
| Sub 2-11 | m/z=270.12 (C₁₈H₁₉Cl=270.80) | Sub 2-12 | m/z=322.15 (C₂₂H₂₃Cl=322.88) |
| Sub 2-13 | m/z=202.05 (C₁₃H₁₁Cl=202.68) | Sub 2-14 | m/z=304.10 (C₂₁H₁₇Cl=304.82) |
| Sub 2-15 | m/z=294.03 (C₁₈H₁₁ClO=294.80) | Sub 2-16 | m/z=264.07 (C₁₈H₁₃Cl=264.75) |
| Sub 2-17 | m/z=245.10 (C₁₆H₄D₇Cl=245.76) | Sub 2-18 | m/z=428.13 (C₃₁H₂₁Cl=428.96) |
| Sub 2-19 | m/z=216.07 (C₁₄H₁₃Cl=216.76) | Sub 2-20 | m/z=278.05 (C₁₈H₁₁ClO=278.74) |
| Sub 2-21 | m/z=428.13 (C₃₁H₂₁Cl=428.96) | Sub 2-22 | m/z=353.10 (C₂₄H₁₆ClN=353.85) |
| Sub 2-23 | m/z=353.10 (C₂₄H₁₆ClN=353.85) | Sub 2-24 | m/z=270.12 (C₁₈H₁₉Cl=270.80) |
| Sub 2-25 | m/z=340.10 (C₂₄H₁₇Cl=340.85) | Sub 2-26 | m/z=238.05 (C₁₆H₁₁Cl=238.71) |
| Sub 2-27 | m/z=197.10 (C₁₂D₉Cl=197.71) | Sub 2-28 | m/z=304.10 (C₂₁H₁₇Cl=304.82) |
| Sub 2-29 | m/z=304.10 (C₂₁H₁₇Cl=304.82) | Sub 2-30 | m/z=340.10 (C₂₄H₁₇Cl=340.85) |

### I. Synthesis of Final Product

### 1. Synthesis example of P-3

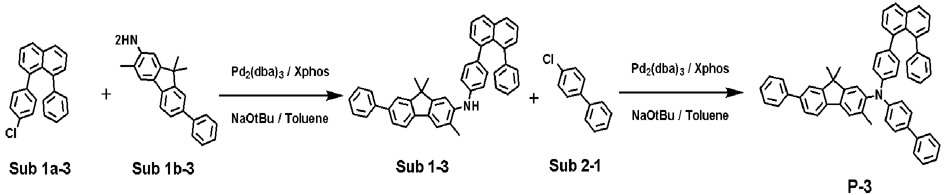

### 1) Synthesis of Sub 1-3

Sub 1a-3 (11.5 g, 36.5 mmol), Sub 1b-3 (12.0 g, 40.2 mmol), Pd₂(dba)₃ (1.0 g, 1.1 mmol), Xphos (8.7 g, 18.3 mmol), NaOt-Bu (7.0 g, 73.1 mmol), toluene (122 mL) were added to a round bottom flask and stirred at 60°C for 4 hours.

After the reaction was completed, the mixture was extracted with toluene and water, the organic layer was dried over MgSO₄ and concentrated, and the resulting compound was recrystallized using a silicagel column to obtain 13.7 g of the product (yield: 65%).

### 2) Synthesis of P-3

Sub 1-3 (13.3 g, 23.0 mmol) was dissolved in toluene (77 mL) in a round-bottom flask, then Sub 2-1 (4.3 g, 23.0 mmol), Pd₂(dba)₃ (0.6 g, 0.7 mmol), Xphos (5.5 g, 11.5 mmol), NaO*t*-Bu (4.4 g, 46.0 mmol) was added and stirred at 100°C for 6 hours. After the reaction was completed, the mixture was extracted with toluene and water, the organic layer was dried over MgSO₄ and concentrated, and the resulting compound was recrystallized using a silicagel column to obtain 9.9 g of the product (yield: 59%).

### 2. Synthesis example of P-14

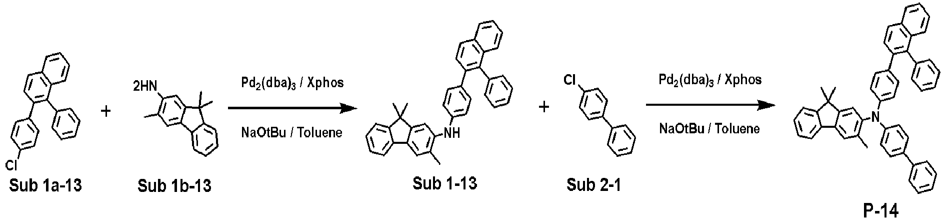

### 1) Synthesis of Sub 1-3

Sub 1-a13 (10.8 g, 34.3 mmol, Sub 1b-13 (11.3 g, 37.7 mmol), Pd₂(dba)₃ (0.9 g, 1.0 mmol), Xphos (8.2 g, 17.2 mmol), NaOt-Bu (6.6 g, 68.6 mmol), Toluene (114 mL) were added to a round-bottom flask, and 11.9 g of the product (yield: 65%) was obtained using the synthetic method of Sub 1-3.

### 2) Synthesis of P-14

Sub 1-13 (11.8 g, 23.5 mmol), Sub 2-1 (4.4 g, 23.5 mmol), Pd₂(dba)₃ (0.6 g, 0.6 mmol), Xphos (5.6 g, 11.8 mmol), NaOt-Bu (4.5 g, 47.0 mmol), Toluene (78 mL) were added to a round-bottom flask, and 10.6 g of the product (yield: 62%) was obtained using the synthetic method of P-3.

### 3. Synthesis example of P-29

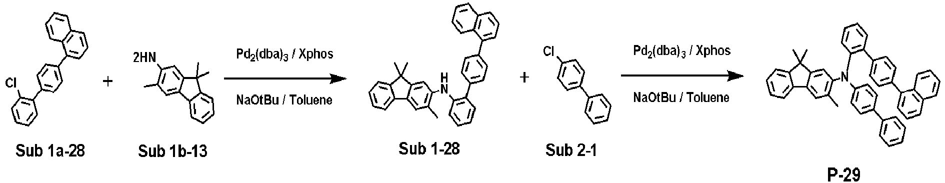

### 1) Synthesis of Sub 1-28

Sub 1-a28 (12.7 g, 40.3 mmol), Sub 1b-13 (9.9 g, 44.4 mmol), Pd₂(dba)₃ (1.1 g, 1.2 mmol), Xphos (9.6 g, 20.2 mmol), NaOt-Bu (7.8 g, 80.7 mmol), Toluene (134 mL) were added to a round-bottom flask, and 13.6 g of the product (yield: 67%) was obtained using the synthetic method of Sub 1-3.

### 2) Synthesis of P-29

Sub 1-28 (12.8 g, 25.5 mmol), Sub 2-1 (4.8 g, 25.5 mmol), Pd₂(dba)₃ (0.7 g, 0.8 mmol), Xphos (6.1 g, 12.8 mmol), NaOt-Bu (4.9 g, 51.0 mmol), Toluene (85 mL) were added to a round-bottom flask, and 11.2 g of the product (yield: 60%) was obtained using the synthetic method of P-3.

### 4. Synthesis example of P-41

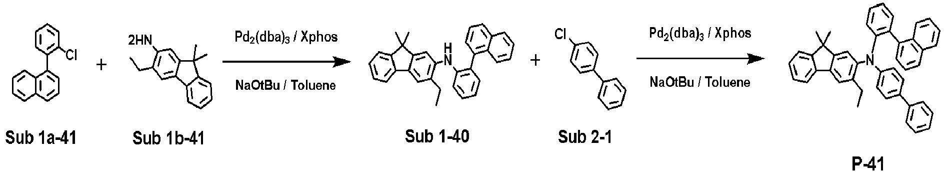

### 1) Synthesis of Sub 1-40

Sub 1-a41 (11.5 g, 48.2 mmol), Sub 1b-41 (12.6 g, 53.0 mmol), Pd₂(dba)₃ (1.3 g, 1.4 mmol), Xphos (11.5 g, 24.1 mmol), NaOt-Bu (9.3 g, 96.4 mmol), Toluene (161 mL) were added to a round-bottom flask, and 15.0 g of the product (yield: 71%) was obtained using the synthetic method of Sub 1-3.

### 2) Synthesis of P-41

Sub 1-40 (14.1 g, 32.1 mmol), Sub 2-1 (6.1 g, 32.1 mmol), Pd₂(dba)₃ (0.9 g, 1.0 mmol), Xphos (7.6 g, 16.0 mmol), NaO*t*-Bu (6.2 g, 64.1 mmol), Toluene (107 mL) were added to a round-bottom flask, and 11.0 g of the product (yield: 58%) was obtained using the synthetic method of P-3.

### 5. Synthesis example of P-64

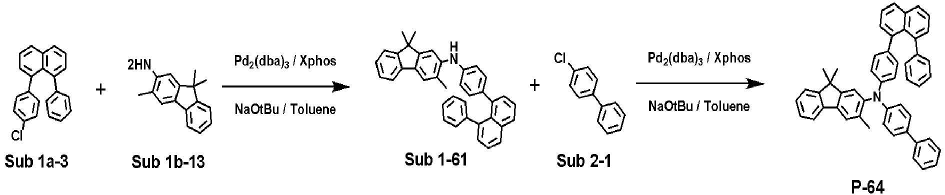

### 1) Synthesis of Sub 1-61

Sub 1-a3 (10.5 g, 33.4 mmol), Sub 1b-13 (8.2 g, 36.7 mmol), Pd₂(dba)₃ (0.9 g, 1.0 mmol), Xphos (8.0 g, 16.7 mmol), NaOt-Bu (6.4 g, 66.7 mmol), Toluene (111 mL) were added to a round-bottom flask, and 12.4 g of the product (yield: 74%) was obtained using the synthetic method of Sub 1-3.

### 2) Synthesis of P-64

Sub 1-61 (12.0 g, 23.9 mmol), Sub 2-1 (4.5 g, 23.9 mmol), Pd₂(dba)₃ (0.7 g, 0.8 mmol), Xphos (5.7 g, 12.0 mmol), NaOt-Bu (4.6 g, 47.8 mmol), Toluene (80 mL) were added to a round-bottom flask, and 9.7 g of the product (yield: 62%) was obtained using the synthetic method of P-3.

### 6. Synthesis example of P-72

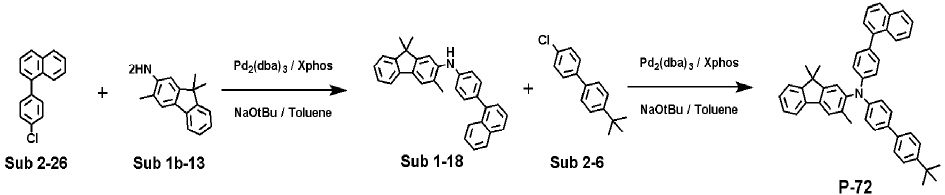

### 1) Synthesis of Sub 1-18

Sub 2-26 (12.2 g, 51.1 mmol), Sub 1b-13 (12.6 g, 56.2 mmol), Pd₂(dba)₃ (1.4 g, 1.5 mmol), Xphos (12.2 g, 25.6 mmol), NaOt-Bu (9.8 g, 102.2 mmol), Toluene (170 mL) were added to a round-bottom flask, and 15.4 g of the product (yield: 71%) was obtained using the synthetic method of Sub 1-3.

### 2) Synthesis of P-72

Sub 1-18 (14.2 g, 33.4 mmol), Sub 2-6 (8.2 g, 33.4 mmol), Pd₂(dba)₃ (0.9 g, 1.0 mmol), Xphos (8.0 g, 16.7 mmol), NaO*t*-Bu (6.4 g, 66.7 mmol), Toluene (111 mL) were added to a round-bottom flask, and 13.7 g of the product (yield: 65%) was obtained using the synthetic method of P-3.

### 7. Synthesis example of P-80

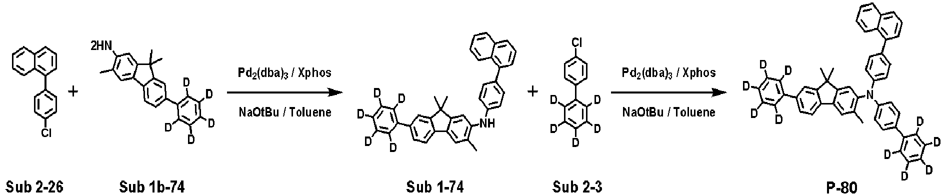

### 1) Synthesis of Sub 1-74

Sub 2-26 (12.3 g, 51.5 mmol), Sub 1b-74 (17.3 g, 56.7 mmol), Pd₂(dba)₃ (1.4 g, 1.5 mmol), Xphos (12.3 g, 25.8 mmol), NaO*t*-Bu (9.9 g, 103.1 mmol), Toluene (172 mL) were added to a round-bottom flask, and 14.4 g of the product (yield: 55%) was obtained using the synthetic method of Sub 1-3.

### 2) Synthesis of P-80

Sub 1-74 (13.4 g, 26.4 mmol, Sub 2-3 (5.1 g, 26.4 mmol), Pd₂(dba)₃ (0.7 g, 0.8 mmol), Xphos (6.3 g, 13.2 mmol), NaO*t*-Bu (5.1 g, 52.9 mmol), Toluene (88 mL) were added to a round-bottom flask, and 17.6 g of the product (yield: 66%) was obtained using the synthetic method of P-3.

Meanwhile, the FD-MS values of compounds P-1 to P-80 of the present invention manufactured according to the above-described synthetic examples are as shown in Table 3.

**[Table 3]**

| compound | FD-MS | compound | FD-MS |
|---|---|---|---|
| P-1 | m/z=659.36 (C₅₀H₄₅N=659.92) | P-2 | m/z=581.30 (C₄₄H₃₁D₄N=581.80) |
| P-3 | m/z=729.34 (C₅₁H₄₁N=729.79) | P-4 | m/z=667.32 (C₅₁H₄₁N=667.90) |
| P-5 | m/z=734.37 (C₅₆H₃₈D₅N=735.00) | P-6 | m/z=667.32 (C₅₁H₄₁N=667.90) |
| P-7 | m/z=667.32 (C₅₁H₄₁N=667.90) | P-8 | m/z=729.34 (C₅₁H₄₁N=729.79) |
| P-9 | m/z=723.39 (C₅₅H₄₉N=724.00) | P-10 | m/z=667.29 (C₅₀H₃₇NO=667.85) |
| P-11 | m/z=683.26 (C₅₀H₃₇NS=683.91) | P-12 | m/z=667.29 (C₅₀H₃₇NO=667.85) |
| P-13 | m/z=683.26 (C₅₀H₃₇NS=683.91) | P-14 | m/z=653.31 (C₅₀H₃₉N=653.87) |
| P-15 | m/z=729.34 (C₅₁H₄₁N=729.79) | P-16 | m/z=729.34 (C₅₁H₄₁N=729.79) |
| P-17 | m/z=703.32 (C₅₄H₄₁N=703.93) | P-18 | m/z=667.32 (C₅₁H₄₁N=667.90) |
| P-19 | m/z=693.34 (C₅₃H₄₃N=693.93) | P-20 | m/z=757.33 (C₅₇H₄₃NO=757.98) |
| P-21 | m/z=759.30 (C₅₆H₄₁NS=760.01) | P-22 | m/z=805.37 (C₆₂H₄₇N=806.06) |
| P-23 | m/z=759.30 (C₅₆H₄₁NS=760.01) | P-24 | m/z=681.30 (C₅₁H₃₉NO=681.88) |
| P-25 | m/z=857.50 (C₆₅H₆₃N=858.23) | P-26 | m/z=747.39 (C₅₇H₄₉N=748.03) |
| P-27 | m/z=743.32 (C₅₆H₄₁NO=743.95) | P-28 | m/z=769.37 (C₅₉H₄₇N=770.03) |
| P-29 | m/z=653.31 (C₅₀H₃₉N=653.87) | P-30 | m/z=729.34 (C₅₁H₄₁N=729.79) |
| P-31 | m/z=805.37 (C₆₂H₄₇N=806.06) | P-32 | m/z=729.34 (C₅₁H₄₁N=729.79) |
| P-33 | m/z=811.42 (C₆₂H₅₃N=812.11) | P-34 | m/z=829.46 (C₆₃H₅₉N=830.17) |
| P-35 | m/z=735.39 (C₅₆H₄₉N=736.02) | P-36 | m/z=801.43 (C₆₁H₅₅N=802.12) |
| P-37 | m/z=717.34 (C₅₅H₄₃N=717.96) | P-38 | m/z=653.31 (C₅₀H₃₉N=653.87) |
| P-39 | m/z=749.40 (C₅₇H₅₁N=750.04) | P-40 | m/z=697.28 (C₅₁H₃₉NS=697.94) |
| P-41 | m/z=591.29 (C₄₅H₃₇N=591.80) | P-42 | m/z=673.37 (C₅₁H₄₇N=673.94) |
| P-43 | m/z=681.30 (C₅₁H₃₉NO=681.88) | P-44 | m/z=619.32 (C₄₇H₄₁N₂=619.85) |
| P-45 | m/z=748.38 (C₅₆H₄₈N₂=749.01) | P-46 | m/z=757.37 (C₅₈H₄₇N=758.02) |
| P-47 | m/z=695.36 (C₅₃H₄₅N=695.95) | P-48 | m/z=698.37 (C₅₃H₃₄D₇N=698.96) |
| P-49 | m/z=577.28 (C₄₄H₃₅N=577.77) | P-50 | m/z=817.37 (C₆₃H₄₇N=818.08) |
| P-51 | m/z=692.41 (C₅₂H₃₂D₁₁N=692.99) | P-52 | m/z=653.31 (C₅₀H₃₉N=653.87) |
| P-53 | m/z=681.34 (C₅₂H₄₃N=681.92) | P-54 | m/z=743.32 (C₅₆H₄₁NO=743.95) |
| P-55 | m/z=704.41 (C₅₃H₃₆D₉N=705.00) | P-56 | m/z=817.37 (C₆₃H₄₇N=818.08) |
| P-57 | m/z=668.40 (C₅₀H₂₄D₁₅N=668.96) | P-58 | m/z=709.37 (C₅₄H₄₇N=709.98) |
| P-59 | m/z=730.33 (C₅₅H₄₂N₂=730.96) | P-60 | m/z=656.33 (C₅₀H₃₆D₃N=656.89) |
| P-61 | m/z=742.33 (C₅₆H₄₂N₂=742.97) | P-62 | m/z=729.34 (C₅₁H₄₁N=729.79) |
| P-63 | m/z=742.33 (C₅₆H₄₂N₂=742.97) | P-64 | m/z=653.31 (C₅₀H₃₉N=653.87) |
| P-65 | m/z=768.44 (C₅₈H₃₆D₁₁N=769.09) | P-66 | m/z=741.34 (C₅₆H₅₅N=742.06) |
| P-67 | m/z=743.36 (C₅₇H₄₅N=743.99) | P-68 | m/z=683.36 (C₅₂H₄₅N=683.94) |
| P-69 | m/z=745.46 (C₅₆H₅₉N=746.09) | P-70 | m/z=745.46 (C₅₆H₅₉N=746.09) |
| P-71 | m/z=659.35 (C₅₀H₃₃D₆N=659.91) | P-72 | m/z=633.34 (C₄₈H₄₃N=633.88) |
| P-73 | m/z=625.43 (C₄₆H₁₉D₂₀N=625.95) | P-74 | m/z=701.39 (C₅₃H₃₉D₆N=701.99) |
| P-75 | m/z=769.37 (C₅₉H₄₇N=770.03) | P-76 | m/z=769.37 (C₅₉H₄₇N=770.03) |
| P-77 | m/z=653.32 (C₅₀H₃₉N=653.87) | P-78 | m/z=659.36 (C₅₀H₄₅N=659.92) |
| P-79 | m/z=729.34 (C₅₆H₄₃N=729.97) | P-80 | m/z=663.37 (C₅₀H₂₉D₁₀N=663.93) |

Meanwhile, exemplary synthesis examples of the present invention represented by Formula 1 have been described, but these are all based on the Buchwald-Hartwig cross coupling reaction, Miyaura boration reaction, Suzuki cross-coupling reaction, Intramolecular acid-induced cyclization reaction (J. mater. Chem.1999, 9, 2095.), Pd(II)-catalyzed oxidative cyclization reaction (Org. Lett.2011, 13, 5504), and PPh₃-mediated reductive cyclization reaction (J. Org. Chem. 2005, 70, 5014.), and it will be easily understood by those skilled in the art that the reaction proceeds even when other substituents defined in Formula 1 are bonded in addition to the substituents specified in the specific synthesis examples.

### Manufacturing Evaluation of Organic Electroluminescent Devices

### [Example 1] Green Organic Electroluminescent Device (Hole Transport Layer)

Compound P-1 and Compound B of the present invention were used on an ITO layer (anode) formed on a glass substrate, and Compound B was doped at a weight ratio of 98:2 to form a hole injection layer with a thickness of 10 nm. Then, Compound P-1 of the present invention was vacuum-deposited on the hole injection layer with a thickness of 110 nm to form a hole transport layer.

Next, compound C-G was vacuum-deposited on the hole transport layer to a thickness of 10 nm to form an emitting auxiliary layer. Afterwards, compound D-G was used as the host material of the emitting layer and tris(2-phenylpyridine)-iridium (hereinafter abbreviated as 'Ir(ppy)₃') was used as the dopant material, and the dopants were doped at a weight ratio of 90:10 to form an emitting layer with a thickness of 30 nm.

Next, compound E was vacuum-deposited on the emitting layer to form a hole-blocking layer with a thickness of 10 nm, and a mixture of compound F and compound G at a weight ratio of 5:5 was used to form an electron transport layer with a thickness of 30 nm on the hole-blocking layer.

Afterwards, compound G was deposited on the electron transport layer to form an electron injection layer with a thickness of 0.2 nm, and then Al was deposited to form a cathode with a thickness of 150 nm.
compound B :4,4',4"-((1*E*,1'*E*,1"*E*)-cyclopropane-1,2,3-triylidenetris(cyanomethaneylylidene))tris(2,3,5,6-tetrafluorobenzonitrile)
compound C-G : *N*-([1,1'-biphenyl]-2-yl)-*N*-(9,9-dimethyl-9H-fluoren-2-yl)-9,9'-spirobi[fluoren]-2-amine
compound D-G : 5-(3-(4,6-diphenyl-1,3,5-triazin-2-yl)phenyl)-7,7-dimethyl-5,7-dihydroindeno[2,1-*b*]carbazole
compound E : 2-(4'-(9,9-dimethyl-9*H*-fluoren-2-yl)-[1,1'-biphenyl]-3-yl)-4,6-diphenyl-1,3,5-triazine
compound F : 2,7-bis(4-(4,6-diphenyl-1,3,5-triazin-2-yl)phenyl)naphthalene
compound G : (8-quinolinolato)lithium

### [Example 2] to [Example 17] Green organic electroluminescent device (hole transport layer)

An organic electroluminescent device was manufactured in the same manner as in Example 1, except that the compound of the present invention described in Table 4 was used instead of the compound P-1 of the present invention as a hole transport layer material.

### [Comparative Example 1] to [Comparative Example 3]

An organic electroluminescent device was manufactured in the same manner as Example 1, except that Comparative Compounds 1 to 3 described in Table 4 were used instead of Compound P-1 of the present invention as a hole transport layer material.

### <comparative compound 1> <comparative compound 2> <comparative compound 3>

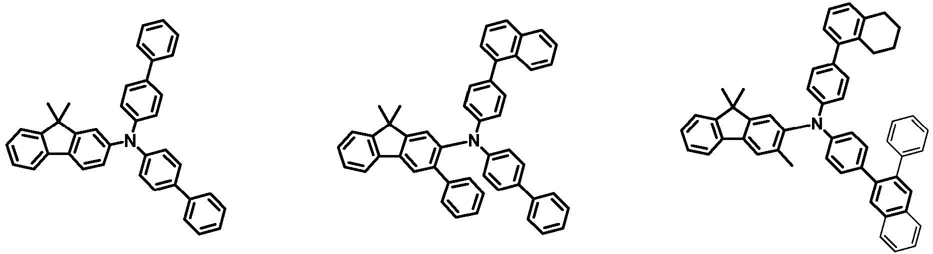

The electroluminescence (EL) characteristics were measured using PR-650 from Photoresearch by applying a forward bias DC voltage to the organic electroluminescence devices manufactured by the examples and comparative examples manufactured by Examples 1 to 17 of the present invention and Comparative Examples 1 to 3. As a result of the measurement, the T95 lifespan was measured using a lifespan measuring device manufactured by Max Science at a standard brightness of 5000 cd/m². Table 4 shows the results of the device fabrication and evaluation.

The measuring apparatus can evaluate the performance of new materials compared to comparative compounds under identical conditions, without being affected by possible daily fluctuations in deposition rate, vacuum quality or other parameters. During the evaluation, one batch contains 4 identically prepared OLEDs including a comparative compound, and the performance of a total of 12 OLEDs is evaluated in 3 batches, so the value of the experimental results obtained in this way indicates statistical significance.

**[Table 4]**

| | compound | Voltage (V) | Current Density (mA/cm²) | Brightness (cd/m²) | Efficiency (cd/A) | Lifetime T(95) |
|---|---|---|---|---|---|---|
| comparative example(1) | comparative compound1 | 5.4 | 13.0 | 5000.0 | 38.6 | 84.2 |
| comparative example(2) | comparative compound2 | 5.3 | 12.7 | 5000.0 | 39.4 | 86.5 |
| comparative example(3) | comparative compound3 | 5.2 | 12.1 | 5000.0 | 41.3 | 85.2 |
| example(1) | P-2 | 4.5 | 11.0 | 5000.0 | 45.6 | 116.2 |
| example(2) | P-4 | 4.4 | 10.5 | 5000.0 | 47.5 | 115.1 |
| example(3) | P-6 | 4.3 | 10.4 | 5000.0 | 47.9 | 119.1 |
| example(4) | P-9 | 4.4 | 10.4 | 5000.0 | 48.1 | 117.3 |
| example(5) | P-12 | 4.4 | 10.4 | 5000.0 | 48.2 | 116.5 |
| example(6) | P-19 | 4.3 | 10.5 | 5000.0 | 47.7 | 117.8 |
| example(7) | P-26 | 4.4 | 10.8 | 5000.0 | 46.2 | 119.9 |
| example(8) | P-29 | 4.4 | 10.6 | 5000.0 | 47.4 | 116.1 |
| example(9) | P-36 | 4.4 | 11.1 | 5000.0 | 45.0 | 118.5 |
| example(10) | P-44 | 4.5 | 10.7 | 5000.0 | 46.6 | 115.6 |
| example(11) | P-48 | 4.4 | 10.2 | 5000.0 | 48.8 | 116.8 |
| example(13) | P-49 | 4.3 | 10.6 | 5000.0 | 47.4 | 118.6 |
| example(14) | P-51 | 4.3 | 10.8 | 5000.0 | 46.1 | 119.3 |
| example(14) | P-55 | 4.5 | 10.8 | 5000.0 | 46.3 | 118.9 |
| example(15) | P-57 | 4.5 | 10.9 | 5000.0 | 45.7 | 117.9 |
| example(16) | P-65 | 4.3 | 10.3 | 5000.0 | 48.5 | 118.6 |
| example(17) | P-73 | 4.4 | 10.9 | 5000.0 | 46.0 | 117.5 |

As can be seen from the results in Table 4, when a green organic electroluminescence device is manufactured using the material for an organic electroluminescence device of the present invention as a hole transport layer material, it can be seen that, compared to the case where comparative compounds 1 to 3 having a similar basic skeleton to the compound of the present invention are used, not only can the driving voltage of the organic electroluminescence device be lowered, but also the luminescence efficiency and lifespan can be improved.

Comparative compounds 1 to 3 and the compound of the present invention have a similar structure in which an arylamine group is bonded to a fluorene group, but there is a difference in the presence or absence of a secondary substituent bonded to a specific position of the fluorene group and the substituent of the amine group.

It can be seen that the device performance of Comparative Compound 2, in which an amine group is bonded to the 2nd position of the fluorene group and phenyl is additionally substituted at the 3rd position, is improved compared to Comparative Compound 1, in which only an amine group is bonded to the 2nd position of the fluorene group and no additional substituents are bonded. Also, in the case of comparative compound 3, it is similar to comparative compound 2, but a methyl group is substituted at position 3, and it is characterized by having an additional substituent in which a specific aliphatic ring group is fused. Comparative compound 3 showed slightly improved driving voltage and efficiency, but its lifespan was reduced due to the fused aliphatic ring group.

Meanwhile, it can be confirmed that the device results of Examples 1 to 17 produced with the compound of the present invention, characterized in that an amine group including a specific aryl moiety is bound to the 2-position of the fluorene core and an alkyl group is bound to the 3-position of the fluorene core, exhibit significantly superior results, and it can be confirmed that the compound of the present invention represented by Formula 1 has superior device performance than other comparative compounds not described in the present specification.

In conclusion, even if the molecular components are similar, the properties of the compound, such as hole characteristics, light efficiency characteristics, energy level, hole injection and mobility characteristics, charge balance of holes and electrons, volume density, and intermolecular distance, can be significantly and unpredictably different depending on the presence or absence of substituted substituents and the substitution position, and it also suggests that the performance of the device can vary due to complex factors rather than a single component affecting the results of the entire device.

In the case of a hole transport layer, it is necessary to understand the relationship with the emitting layer (host), and even if a similar core is used, it would be very difficult for even a person skilled in the art to infer the characteristics exhibited by the hole transport layer using the compound according to the present invention.

Moreover, although the device characteristics in which the compound of the present invention is applied to only one layer of the hole transport layer were described in the evaluation results of the aforementioned device fabrication, the compound of the present invention can be used by applying it to both the hole transport layer and the emitting-auxiliary layer.

Although exemplary embodiments of the present invention have been described for illustrative purposes, those skilled in the art will appreciate that various modifications, additions and substitutions are possible, without departing from the scope and spirit of the invention as disclosed in the accompanying claims. Therefore, the embodiment disclosed in the present invention is intended to illustrate the scope of the technical idea of the present invention, and the scope of the present invention is not limited by the embodiment.

The scope of the present invention shall be construed on the basis of the accompanying claims, and it shall be construed that all of the technical ideas included within the scope equivalent to the claims belong to the present invention.

### [Description of the numerals]

| | | | |
|---|---|---|---|
| 100, 200, 300: | organic electronic element | 110 : | the first electrode |
| 120 : | hole injection layer | 130 : | hole transport layer |
| 140 : | emitting layer | 150 : | electron transport layer |
| 160 : | electron injection layer | 170 : | second electrode |
| 180 : | light efficiency enhancing Layer | 210 : | buffer layer |
| 220 : | emitting auxiliary layer | 320 : | first hole injection layer |
| 330 : | first hole transport layer | 340 : | first emitting layer |
| 350 : | first electron transport layer | 360 : | first charge generation layer |
| 361 : | second charge generation layer | 420 : | second hole injection layer |
| 430 : | second hole transport layer | 440 : | second emitting layer |
| 450 : | second electron transport layer | CGL : | charge generation layer |
| ST1 : | first stack | ST2 : | second stack |

## Claims

1. A compound represented by Formula 1: 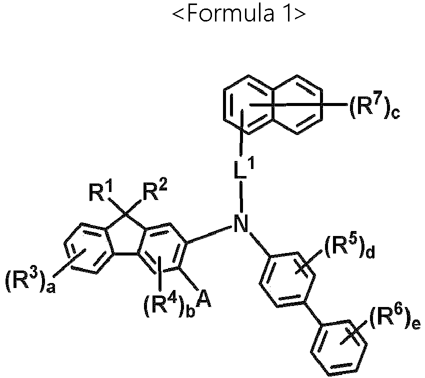 Wherein:
R¹ and R² are the same or different from each other and are independently C₁-C₅₀ alkyl groups substituted or unsubstituted with deuterium;
R³, R⁴, R⁵, R⁶ and R⁷ are the same or different from each other and are independently selected from the group consisting of a hydrogen; deuterium; a C₆-C₆₀ aryl group; a fluorenylene group; a C₂-C₆₀ heterocyclic group including at least one heteroatom of O, N, S, Si or P; and a fused ring group of a C₃-C₆₀ aliphatic ring and a C₆-C₆₀ aromatic ring; a C₁-C₅₀ alkyl group; a C₂-C₂₀ alkenyl group; a C₂-C₂₀ alkynyl group; a C₁-C₃₀ alkoxyl group; and a C₆-C₃₀ aryloxy group; and a C₃-C₆₀ aliphatic ring; or may be bonded to adjacent groups to form an aromatic ring or a heteroaromatic ring,
A is an a C₁-C₅₀ alkyl group;
L¹ is each independently selected from the group consisting of a C₆-C₆₀ arylene group;
a fluorenylene group; a C₂-C₆₀ heteroarylene group including at least one heteroatom of O, N, S, Si or P; a C₃-C₆₀ aliphatic ring; and a fused ring group of a C₃-C₆₀ aliphatic ring and a C₆-C₆₀ aromatic ring;
a and d are independently integers from 0 to 4, b is an integer from 0 to 2, c is an integer from 0 to 7, and e is an integer from 0 to 5,
wherein the aryl group, arylene group, heteroarylene group, heterocyclic group, fluorenyl group, fluorenylene group, aliphatic ring group, fused ring group, alkyl group, alkenyl group, alkynyl group, alkoxyl group and aryloxy group may be further substituted with one or more substituents selected from the group consisting of deuterium; halogen; silane group; siloxane group; boron group; germanium group; cyano group; nitro group; C₁-C₂₀ alkylthio group; C₁-C₂₀ alkoxyl group; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group; a C₃-C₂₀ aliphatic ring; a C₇-C₂₀ arylalkyl group; a C₈-C₂₀ arylalkenyl group; and a C₇-C₂₀ alkylaryl group; also the hydrogen of these substituents may be further substituted with one or more deuterium, and also the substituents may be bonded to each other to form a saturated or unsaturated ring, wherein the term 'ring' means a C₃-C₆₀ aliphatic ring or a C₆-C₆₀ aromatic ring or a C₂-C₆₀ heterocyclic group or a fused ring formed by the combination thereof.

2. The compound according to claim 1, wherein Formula 1 may be represented by Formulas 1-1 or 1-2: 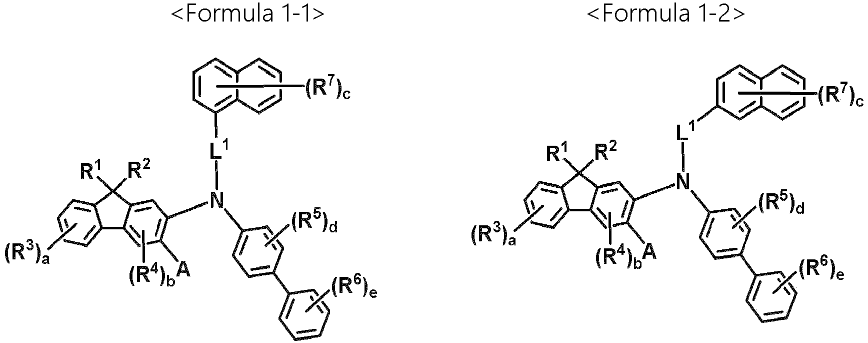 Wherein, R¹, R², R³, R⁴, R⁵, R⁶, R⁷, L¹, A, a, b, c, d and e are the same as defined in claim 1.

3. The compound according to claim 1, wherein A is represented by any one of the following Formulas A-1 to A-3: 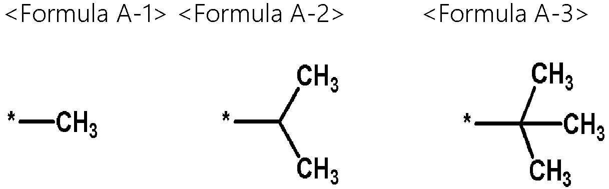 Wherein, hydrogen can be further substituted with one or more deuterium.

4. The compound according to claim 1, wherein L¹ is represented by any one of the following Formulas L-1 to L-6: 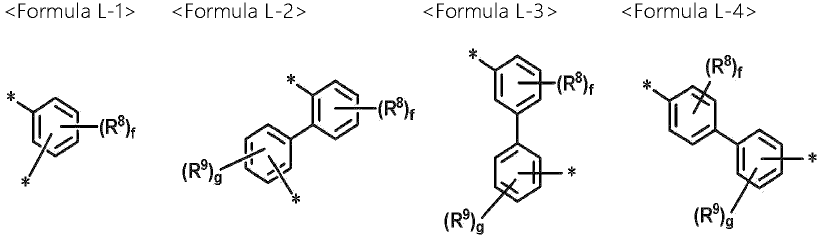 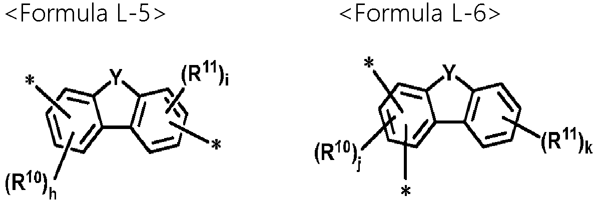 Wherein:
R⁸, R⁹, R¹⁰ and R¹¹ are each the same or different, and each independently selected from the group consisting of a hydrogen; deuterium; halogen; cyano group; nitro group; C₁-C₂₀ alkoxyl group; C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group including at least one heteroatom of O, N, S, Si or P; C₃-C₂₀ aliphatic group; C₇-C₂₀ arylalkyl group; C₈-C₂₀ arylalkenyl group; and a C₇-C₂₀ alkylaryl group;
or an adjacent plurality of R⁸s or plurality of R⁹s or plurality of R¹⁰s or plurality of R¹¹s may be bonded to each other to form a ring,
Y is O, S, NR or CR'R",
Wherein R, R', R" are each independently selected from the group consisting of an C₁-C₂₀ alkyl group; C₂-C₂₀ alkenyl group; C₂-C₂₀ alkynyl group; C₆-C₂₀ aryl group; C₆-C₂₀ aryl group substituted with deuterium; a fluorenyl group; C₂~C₂₀ heterocyclic group including at least one heteroatom of O, N, S, Si or P; C₃-C₂₀ aliphatic group; and a fused ring group of a C₃-C₂₀ aliphatic ring and a C₆-C₂₀ aromatic ring;
f, g and k are independently integers from 0 to 4, h and i are independently integers from 0 to 3, j is an integer from 0 to 2,
* indicates the position to be bonded.

5. The compound according to claim 1, wherein the compound represented by Formula 1 may be any one of the following compounds P-1 to P-80:

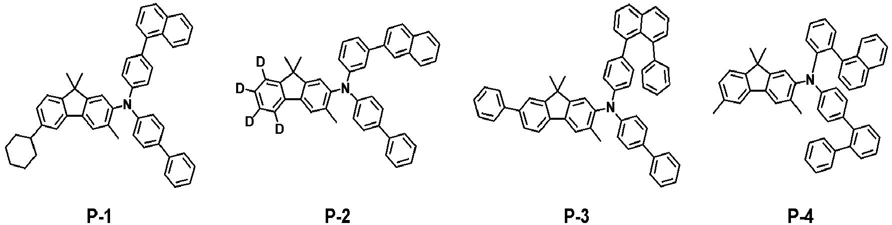

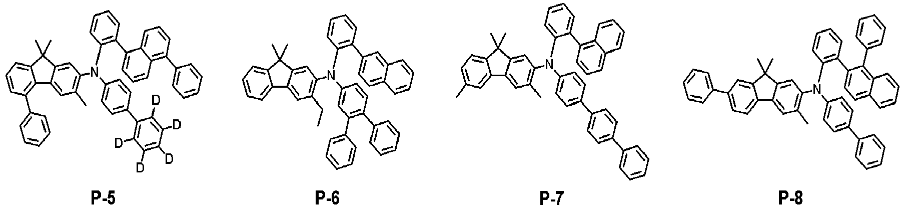

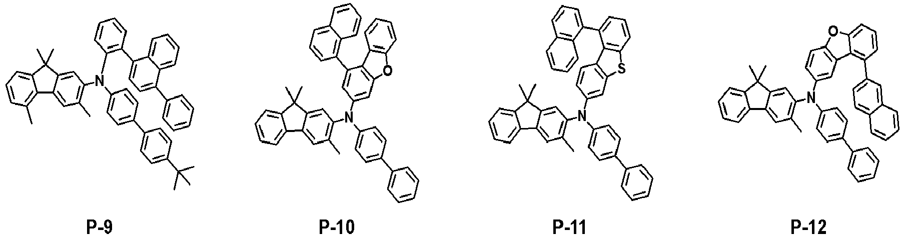

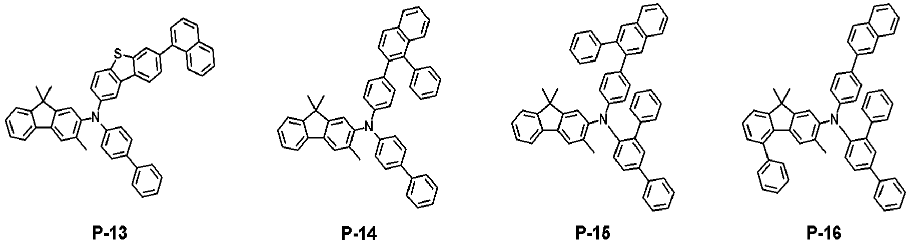

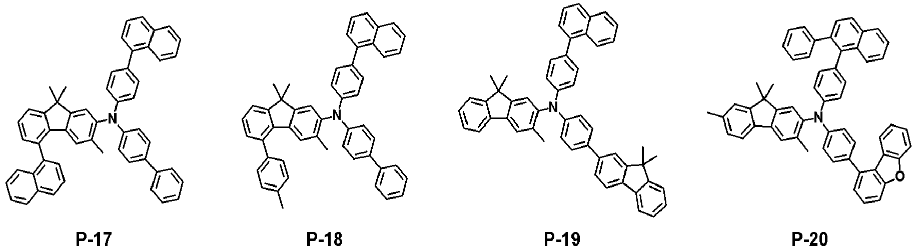

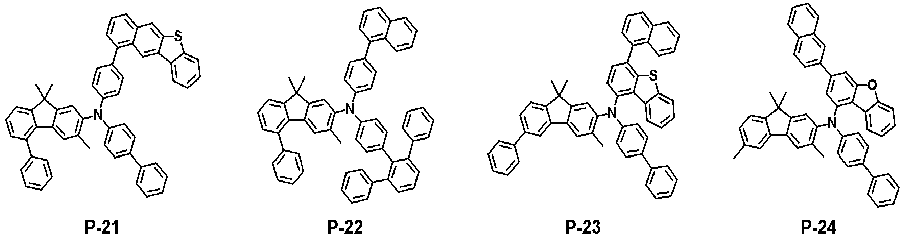

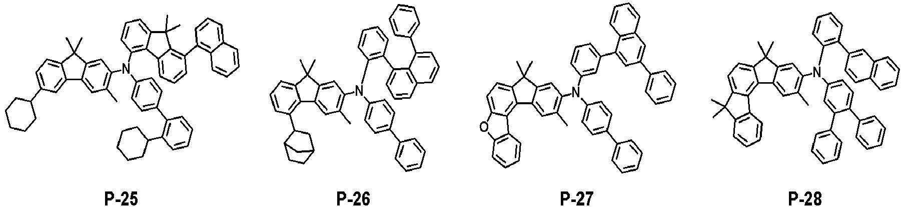

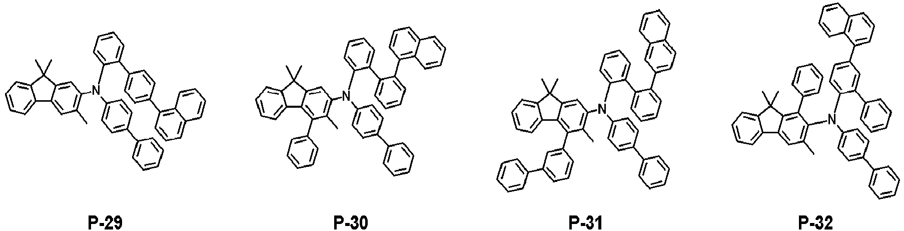

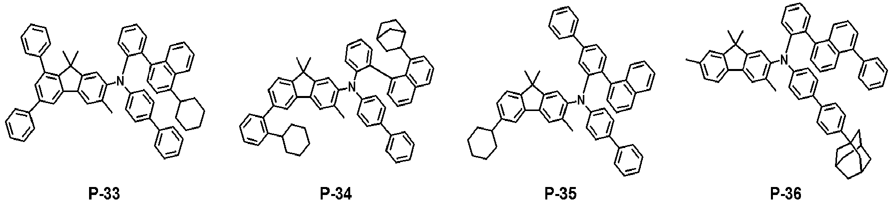

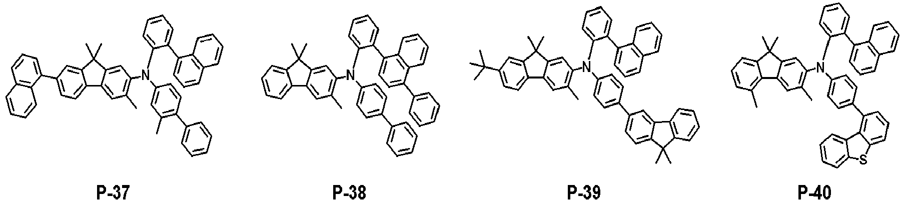

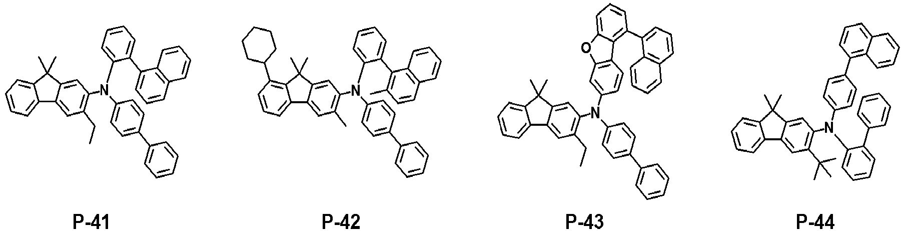

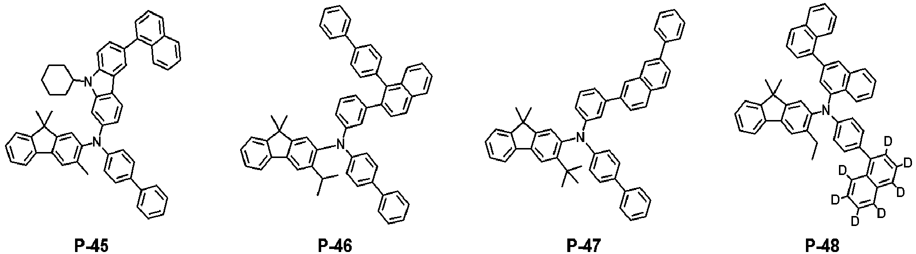

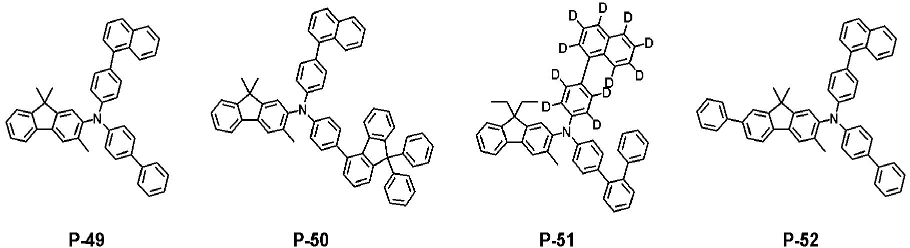

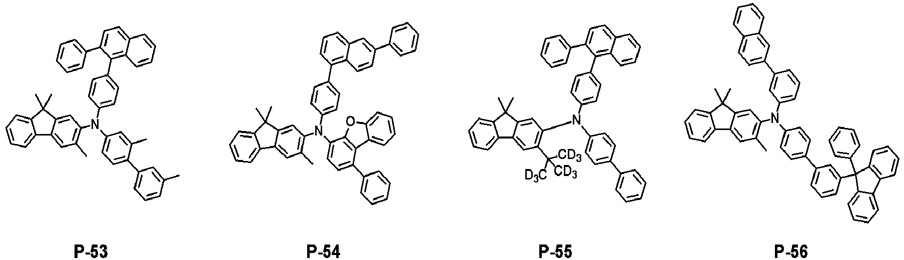

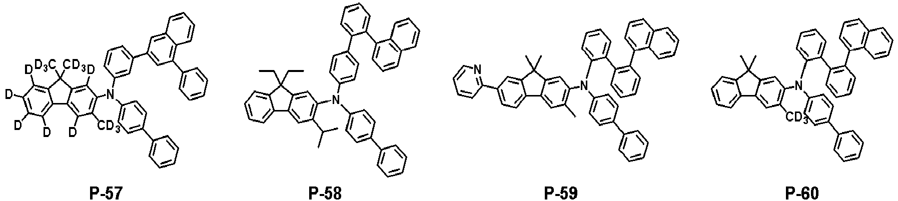

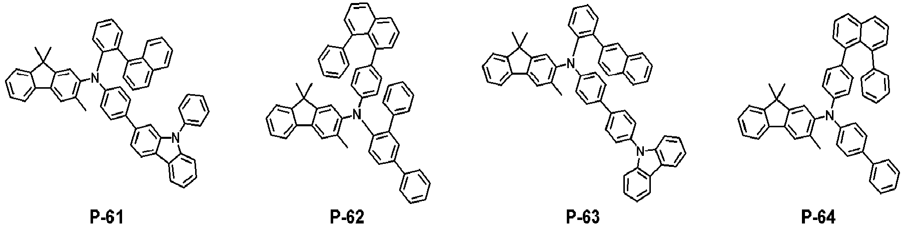

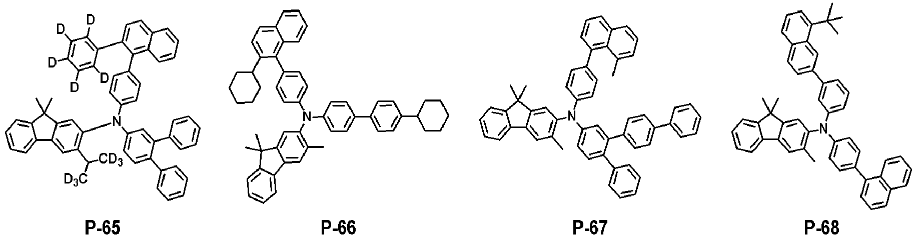

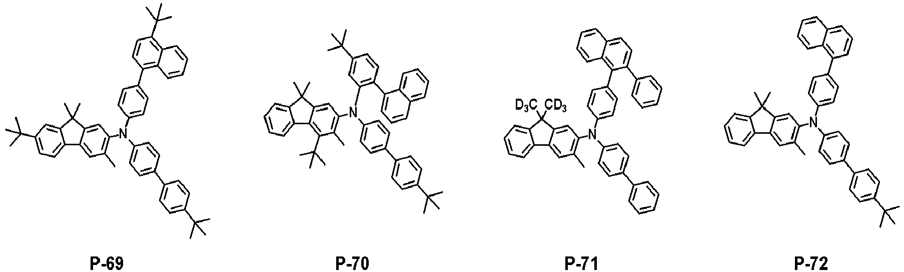

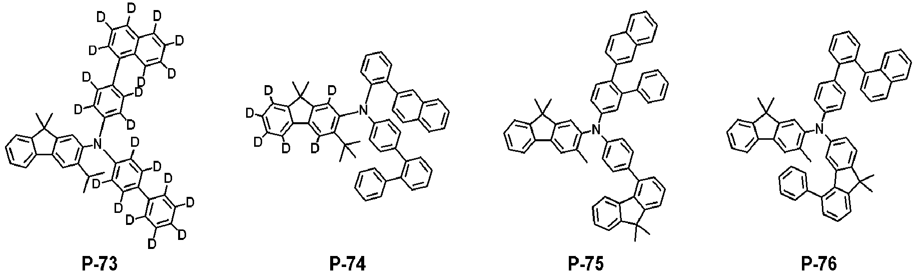

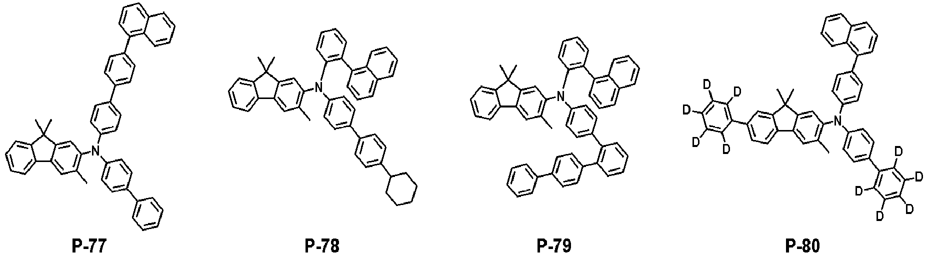

6. An organic electronic element comprising an anode, a cathode, and an organic material layer formed between the anode and the cathode, wherein the organic material layer comprises a single compound or 2 or more compounds represented by Formula 1 of claim 1.

7. The organic electronic element according to claim 6, wherein the organic material layer comprises at least one of a hole injection layer, a hole transport layer, an emitting-auxiliary layer, an emitting layer, an electron transport-auxiliary layer, an electron transport layer and an electron injection layer.

8. The organic electronic element according to claim 6, wherein the organic material layer is a hole transport layer.

9. The organic electronic element according to claim 6, wherein the organic material layer comprises 2 or more stacks comprising a hole transport layer, an emitting layer and an electron transport layer sequentially formed on the anode.

10. The organic electronic element according to claim 9, wherein the organic material layer further comprises a charge generation layer formed between the 2 or more stacks.

11. An electronic device comprising a display device comprising the organic electronic element of claim 6; and a control unit for driving the display device.

12. The electronic device according to claim 11, wherein the organic electronic element is at least one of an OLED, an organic solar cell, an organic photo conductor(OPC), organic transistor (organic TFT) and an element for monochromic or white illumination.

13. A method for reusing Formula 1 of claim 1 comprising:
recovering a crude organic light emitting material comprising Formula 1 of claim 1 from a deposition apparatus used in the process for depositing the organic emitting material to prepare an organic an organic light emitting device;
removing impurities from the crude organic light emitting material;
recovering the organic light emitting material after the impurities are removed; and
purifying the recovered organic light emitting material to have a purity of 99.9% or higher.
